# EUROPEAN PATENT SPECIFICATION

(11) **EP 4 226 233 B1**
(45) Date of publication and mention of the grant of the patent: **23.09.2026**
(21) Application number: 21878486.6
(22) Date of filing: 06.10.2021
(51) Int. Cl.: G06N 20/20, G06N 3/045, G06N 20/10, G16H 50/70, G16H 70/00, G06N 3/0442, G06N 3/0455, G06N 3/0464, G06N 3/09, G06N 5/01, G01N 33/50

(54) **METHODS FOR EXPOSOMIC CLINICAL APPLICATIONS**
VERFAHREN FÜR KLINISCHE EXPOSOMALE ANWENDUNGEN
PROCÉDÉS DESTINÉS À DES APPLICATIONS CLINIQUES EXPOSOMIQUES

(30) Priority: 06.10.2020 US 202063088375 P; 04.12.2020 US 202063121792 P; 23.03.2021 US 202163164964 P
(43) Date of publication of application: 16.08.2023
(73) Proprietor: Icahn School of Medicine at Mount Sinai, New York, NY 10029 (US)
(72) Inventor: ARORA, Manish, New York, NY 10029 (US); CURTIN, Paul, New York, NY 10029 (US); AUSTIN, Christine, New York, NY 10029 (US)
(74) Representative: Grund, Martin
(86) International application number: PCT/US2021/053838
(87) International publication number: WO 2022/076603

(56) References cited:
- US-A1- 2017 131 295
- US-A1- 2019 262 831
- US-A1- 2020 063 202
- US-A1- 2020 135 337
- BLANDINE COMTE: "Network and Systems Medicine: Position Paper of the European Collaboration on Science and Technology Action on Open Multiscale Systems Medicine", vol. 3, no. 1, 1 July 2020 (2020-07-01), pages 67 - 90, XP093166254, ISSN: 2690-5949, Retrieved from the Internet <URL:https://www.ncbi.nlm.nih.gov/pmc/articles/PMC7500076/pdf/nsm.2020.0004.pdf> DOI: 10.1089/nsm.2020.0004
- MEGAN M NIEDZWIECKI: "The Exposome: Molecules to Populations", ANNU. REV. PHARMACOL. TOXICOL. 2019. 59:107-27, 10 August 2018 (2018-08-10), XP093166372, Retrieved from the Internet <URL:https://www.annualreviews.org/docserver/fulltext/pharmtox/59/1/annurev-pharmtox-010818-021315.pdf?expires=1716549936&id=id&accname=guest&checksum=6CB4771A06EF7B05B203D6327411A8D9> DOI: 10.1146/annurev-pharmtox
- AUSTIN CHRISTINE ET AL: "Uncovering system-specific stress signatures in primate teeth with multimodal imaging", vol. 6, no. 1, 1 May 2016 (2016-05-01), XP055884415, Retrieved from the Internet <URL:https://www.nature.com/articles/srep18802.pdf> DOI: 10.1038/srep18802
- MAEKAWA MOTOKO ET AL: "Utility of Scalp Hair Follicles as a Novel Source of Biomarker Genes for Psychiatric Illnesses", BIOLOGICAL PSYCHIATRY, ELSEVIER, AMSTERDAM, NL, vol. 78, no. 2, 11 September 2014 (2014-09-11), pages 116 - 125, XP029215883, ISSN: 0006-3223, DOI: 10.1016/J.BIOPSYCH.2014.07.025

## Description

### BACKGROUND

Approximately 50% of all pharmaceutical phase three interventions (including but not limited to clinical trials and other interventional study designs) will fail from a lack of efficacy or adverse effects resulting from the administration of the treatment. One potential explanation to the high failure rate is the intricate and complex biology of the human body that can drastically differ between individuals. Superficial screening criterion and participant eligibility for intervention alone is incapable of capturing the complexity of the complex human biology.

Comte, Network and Systems Medicine, 2020 discloses an approach to link a biomarker profile of a patient to prediction of drug effect.

However, there is an unmet need for improved systems and methods for screening patients with greater detail to optimize interventions, provide targeted pharmaceutical intervention, and predict early onset of disease.

### SUMMARY

The standard for selection criterion of subjects for interventions and resulting administration of commercial pharmaceuticals depends on data that is limited in scope. The selection criteria e.g., weight, gender, chronic diseases, family disease history or even a blood draw is widely accepted in the medical community as the gold standard, yet such meta data is merely an instantaneous snapshot of an individual's unique complex biology that is constantly evolving. With such coarse categorization and classification of individuals by their clinical metadata, there exists undesirable side-effects and complications for the administration of pharmaceutical without a clear understanding or reasoning behind variability between subjects with similar clinical metadata. To improve upon current best practices and developed gold standards, new innovation towards a richer more representative dataset for screening and classifying individuals' must be made.

The present invention addresses these needs by providing a method for evaluating the effects of an intervention in a plurality of subjects, the method comprising:
sampling, for each respective subject in the plurality of subjects, each respective position in a plurality of positions along a reference line on a corresponding biological sample associated with chemical dynamics of the respective subject, thereby obtaining a corresponding plurality of chemical samples for the respective subject, each chemical sample in the plurality of chemical samples corresponding to a different position in the plurality of positions, and each position in the plurality of positions representing a different period of growth of the corresponding biological sample associated with chemical dynamics, wherein the biological sample comprises a tooth sample, a nail sample, a hair sample, or any combination thereof, and wherein the plurality of positions comprises:
one or more positions representing a period of growth prior to the intervention,
one or more positions representing a period of growth during the intervention, and
one or more positions representing a period of growth after the intervention;
analyzing, for each respective subject in the plurality of subjects, the corresponding plurality of chemical samples for the respective subject thereby obtaining a corresponding first dataset that includes a plurality of traces, each trace in the plurality of traces being a concentration of a corresponding chemical, in a plurality of chemicals, over time collectively determined from the plurality of chemical samples,
applying, for each respective subject in the plurality of subjects and for each chemical in the plurality of chemicals, a distributed lag model as a function of time relative to the intervention to the concentration of the respective chemicals measured from the plurality of positions, to generate a corresponding contribution data set representing the contribution of the intervention to the concentration of the respective chemicals in the respective subject as a function of time; and
evaluating changes in chemical dynamics in response to the intervention using the corresponding contribution data set, for each of the one or more chemicals, from each respective subject in the plurality of subjects
wherein the intervention comprises an environmental input, a pharmaceutical compound, a diet, a supplement, a nutraceutical, or any combination thereof, for each respective subject of the plurality of subjects participating in a clinical trial, a community trial, or any combination thereof.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." and "FIGS." herein), of which:
**FIG. 1** shows an overview of the computer implemented exposomic system, as seen in some embodiments.
**FIGS. 2A-B** show the data workflow for training an intervention predictive model using subjects' clinical database data (clinical meta data), exposome biochemical signature, and intervention outcomes **(****FIG. 2A****)** and using the trained predictive model **(****FIG. 2B****)** to, for example, predict a given subject's intervention outcome, as seen in some embodiments.
**FIGS. 3A-B** show the data workflow for training a pharmaceutical and or nutraceutical optimal selection predictive model using subjects' clinical database data (clinical meta data), exposomic features, percent difference between subject's post-treatment exposomic features and reference exposomic features. **FIG. 3A** shows the training workflow to train the predictive model, and **FIG. 3B** shows the use of the trained predictive model, as seen in some embodiments.
**FIG. 4** illustrates a flow diagram for selecting subjects for an intervention based on their exposome biochemical signature profile, as seen in some embodiments.
**FIG. 5** shows a flow diagram for selecting optimal pharmaceutical or nutraceutical treatments based on a comparison of exposomic features of a subjects in comparison to a reference treatment exposomic features, as seen in some embodiments herein.
**FIGS. 6A-D** show exposome biochemical profiles for various exposome signatures (e.g., tin, lead, calcium and magnesium) for a subject that has not received an intervention (blue) and for a subject that has received an intervention (orange, grey, and teal), as seen in some embodiments herein.
**FIGS. 7A-7B** illustrate clustering of one or more subjects' one or more exposome biochemical profiles **(****FIG. 7A****)** and how such clustering of data may be correlated to disease or disorders of the one or more subjects **(****FIG. 7B****),** as seen in some embodiments herein.
**FIG. 8** shows sub-typing of subjects using exposomic features derived from hair analysis. Exposome biochemical signature data were extracted via analytical methods disclosed elsewhere herein. Unsupervised clustering analysis is shown to identify discrete subtypes of patients with autism spectrum disorder, as seen in some embodiments herein.
**FIG. 9** illustrates the systems and methods utilized to collect and analyze geographical temporal dynamics of annotated exposome pathways through a deep data science framework, as seen in some embodiments herein.
**FIG. 10** shows the temporal aspect of obtaining 100 data timepoints from a single biological sample characterizing the dynamics of physiology at different life stages, as seen in some embodiments herein.
**FIG. 11** shows the various chemical signatures and their respective grouping measured by the methods and systems, as seen in some embodiments herein.
**FIG. 12** shows both temporal and spatial immunohistochemistry (IHC) fluorescence data captured by methods and systems described herein. Specifically, the C-reactive protein IHC fluorescence data illustrates a sharp increase in inflammation prior to birth correlated to the development of autism, as seen in some embodiments herein.
**FIG. 13** shows a method of measuring metal chemical biomarkers of teeth and correlating the spatial distribution of the metal chemical biomarkers across teeth growth lines to onset of disease, disease prognosis, disease diagnosis, changes in biochemical physiology, etc., as seen in some embodiments herein.
**FIG. 14** shows machine learning, informatics, and deep learning platform configured to generate robust and generalizable predictive models of disease (e.g., ASD, ADHD, etc.) diagnosis prior to disease onset.
**FIG. 15** shows a method of phenotyping, pathway identification, metabolic phenotyping, and clinical sub-typing of various physiological outcomes by unsupervised pattern recognition of an exposome map.
**FIG. 16** shows impacted probiotic metabolic and corresponding biochemical pathways measured by the methods and systems, as seen in some embodiments herein.
**FIG. 17** shows impacted gluten metabolic and corresponding biochemical pathways measured by the methods and systems, as seen in some embodiments herein.
**FIG. 18** shows the pathway importance weight from a study of over 500 participants with autism utilized by the methods and systems described to recommend pharmaceutical and nutraceutical compounds to treat autism.
**FIGS. 19A-C** show various forms of exposomic signature data representation, as seen in some embodiments herein.
**FIGS. 20A-D** show the comparison of calcium (FIGS. 20A-B) and copper (FIGS. 20C-D) exposomic signatures and their corresponding attractor graphical representations, as seen in some embodiments.
**FIGS. 21A-B** illustrate prenatal recurrence networks for child with neurotypical **(****FIG. 21A****)** and autism spectrum disorder **(****FIG. 21B****),** as seen in some embodiments.
**FIG. 22** illustrates a flow diagram for a method of outputting one or more quantitative metrics of a subject's one or more exposomic signatures, as seen in some embodiments.
**FIG. 23** illustrate a flow diagram of a method for outputting a prediction of one or more subjects' phenotypic data.

### DETAILED DESCRIPTION

The invention is defined by the appended claims.

Approximately 50% of all pharmaceutical phase three interventions will fail from a lack of efficacy or adverse effects of the treatment experienced by the volunteer participants. One potential explanation to the high failure rate is the intricate and complex biology of the human body that can drastically differ between individuals. Superficial screening criterion and eligibility for intervention participants alone is incapable of capturing the complexity of the complex human biology. Therefore, there is an unmet need for systems and methods of screening patients with greater detail to optimize interventions, provide targeted pharmaceutical intervention, and predict early onset of disease.

The standard for selection criterion of subjects for intervention and resulting administration of commercial pharmaceuticals depends on data that is limited in scope. The selection criteria e.g., weight, gender, chronic diseases, family disease history or even a blood draw is widely accepted in the medical community as the gold standard, yet such meta data is merely an instantaneous snapshot of an individual's unique complex biology that is constantly evolving. With such coarse categorization and classification of individuals by their clinical metadata, there exists undesirable side-effects and complications for the administration of pharmaceutical without a clear understanding or reasoning behind variability between subjects with similar clinical metadata. To improve upon current best practices and developed gold standards, new innovation towards a richer more representative dataset for screening and classifying individuals' must be made.

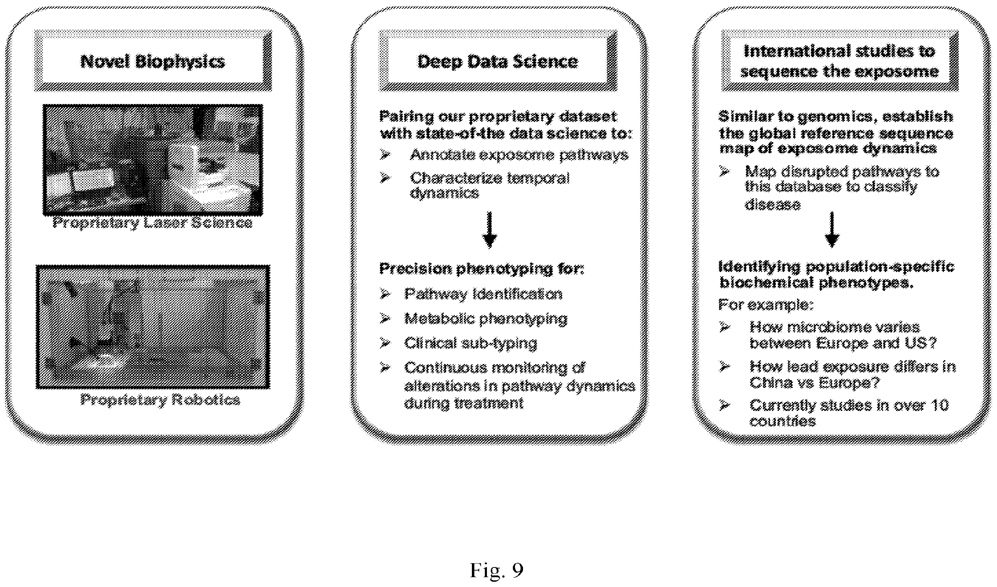

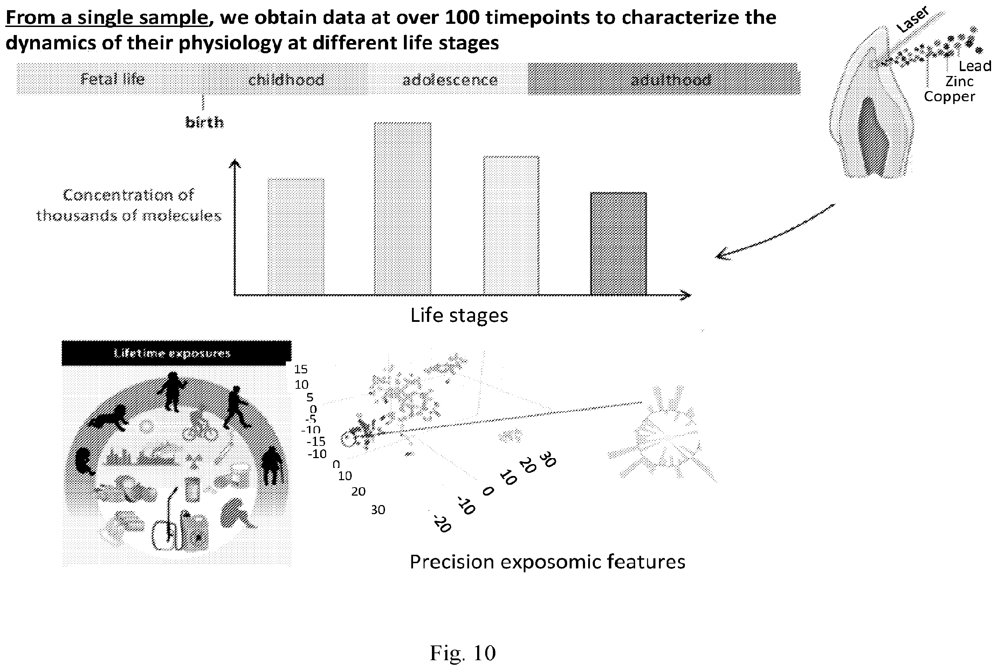

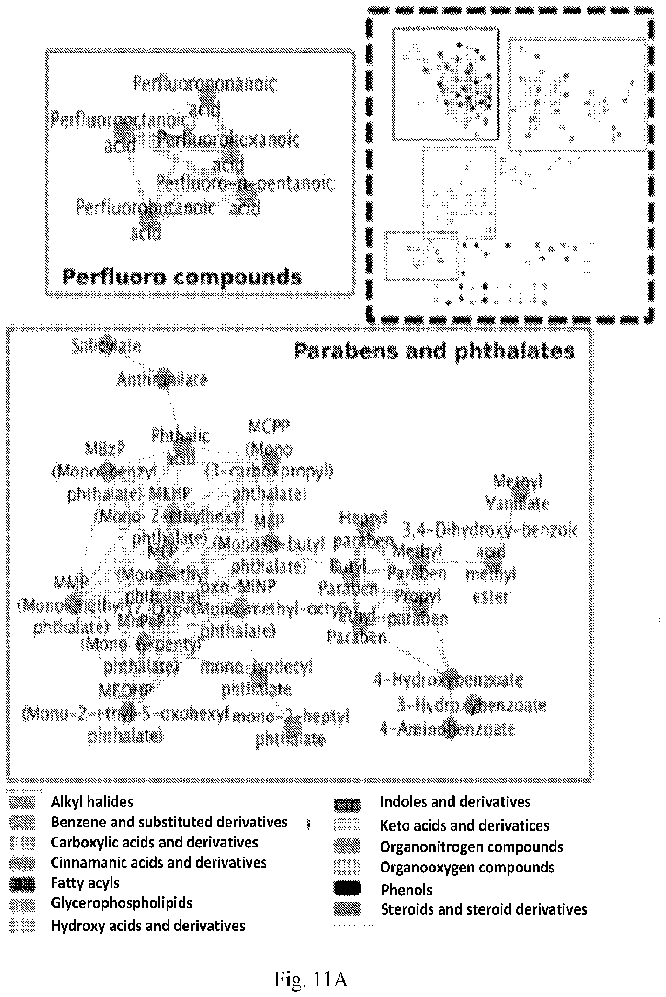

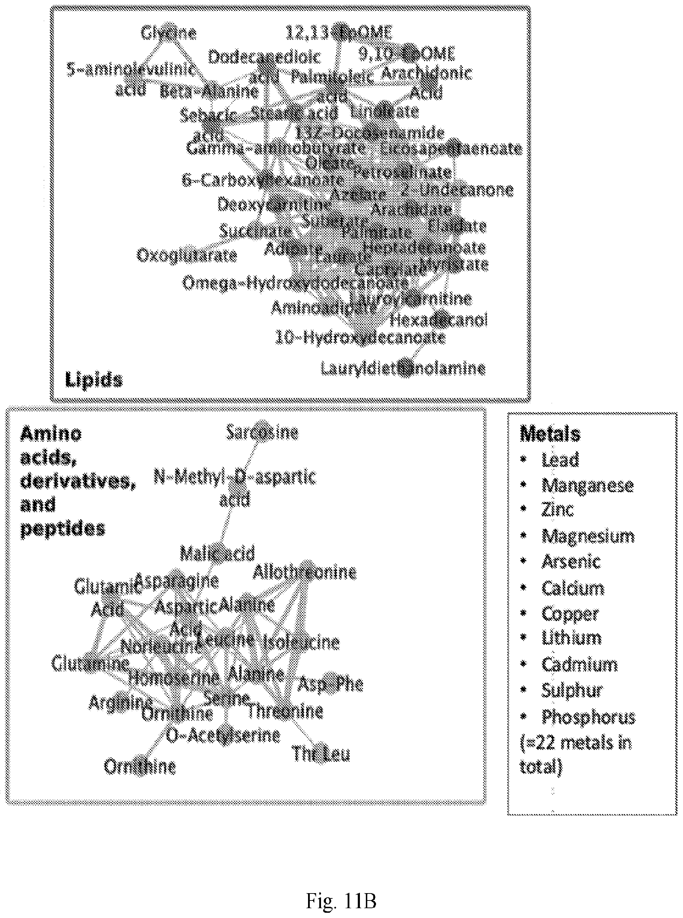

The present disclosure addresses these needs through systems (not claimed as such but useful for understanding the invention) and methods capable of analyzing and classifying subjects by their exposome biochemical signature profile, as shown in **FIG. 9****.** The exposomic feature analysis of the present disclosure may comprise analyzing over 50,000 biochemical signatures **(****FIG. 11****)** in a non-invasive manner from a hair shaft, tooth, finger nail, toe nail, physiologic parameter, or any combination thereof. With such systems and methods of analysis of the present invention, subtle changes in a subject's biochemistry induced by, for example, diet, air-pollution, psychological stress, exposure to pesticides, or industrial chemical **(****FIG. 10****)** to name a few factors, may be investigated and correlated to intervention response outcomes and targeted highly efficacious pharmaceuticals and nutraceuticals.

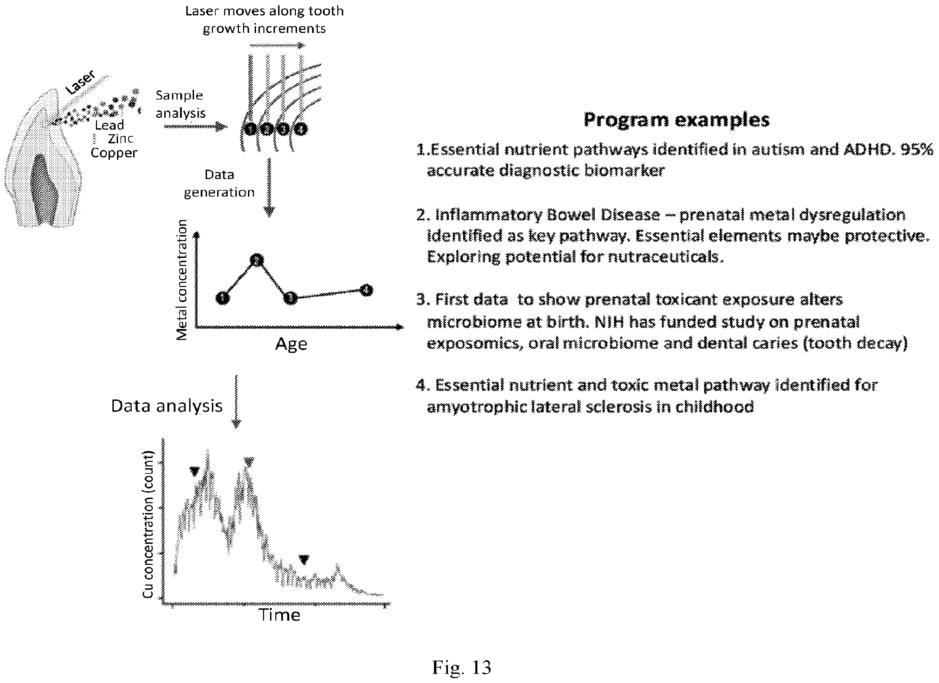

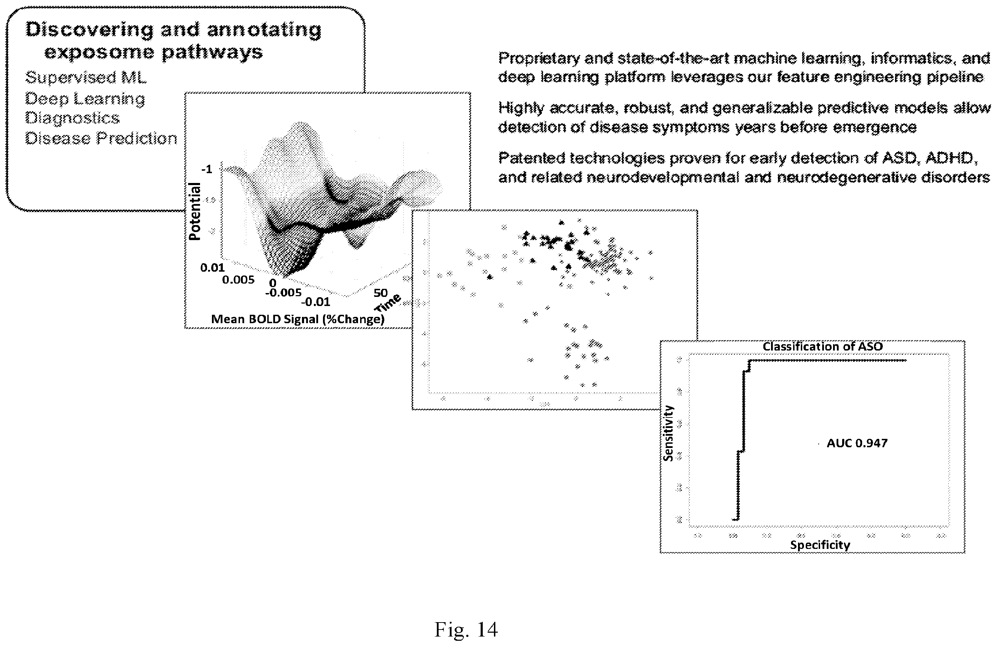

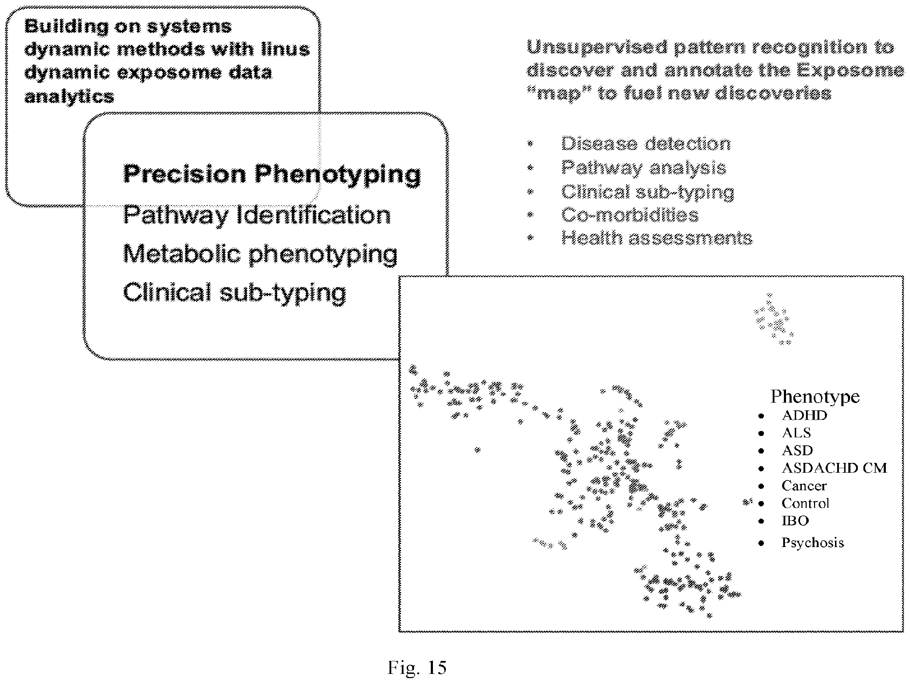

In addition to the copious dataset generated by exposomic biochemical signature analysis, exposomic biochemical signature analysis can also provide insight into temporal fluctuations of said biochemical signatures over the span of a subject's life, as shown in **FIG. 10****.** Such an approach may be utilized to screen individuals suffering from life debilitating diseases to determine what single or combination of exposomic features contribute to the development of the disease. The identified pathways may then be used to train statistical, machine learning, and/or artificial intelligence predictive models capable of predicting early onset of disease from the exposomic features of an otherwise healthy subject at a stage where intervention may provide substantial impact, as seen in **FIGS. 13-15****.**

### Computer Implemented Exposomic System.

In an aspect, disclosed herein, but not claimed isa computer implemented exposomic system for gathering, storing, cataloguing, comparing, analyzing, or any combination thereof, exposomic biochemical signatures for one or more subjects. In some embodiments, the exposomic biochemical signatures may be used at least in part for optimizing selection criterion for subjects participating in interventions. In some embodiments, the exposomic biochemical signatures is used at least in part for suggesting optimal pharmaceutical or nutraceutical treatment for subjects in need thereof. In some embodiments, intervention may comprise a clinical trial, community trial, or any combination thereof.

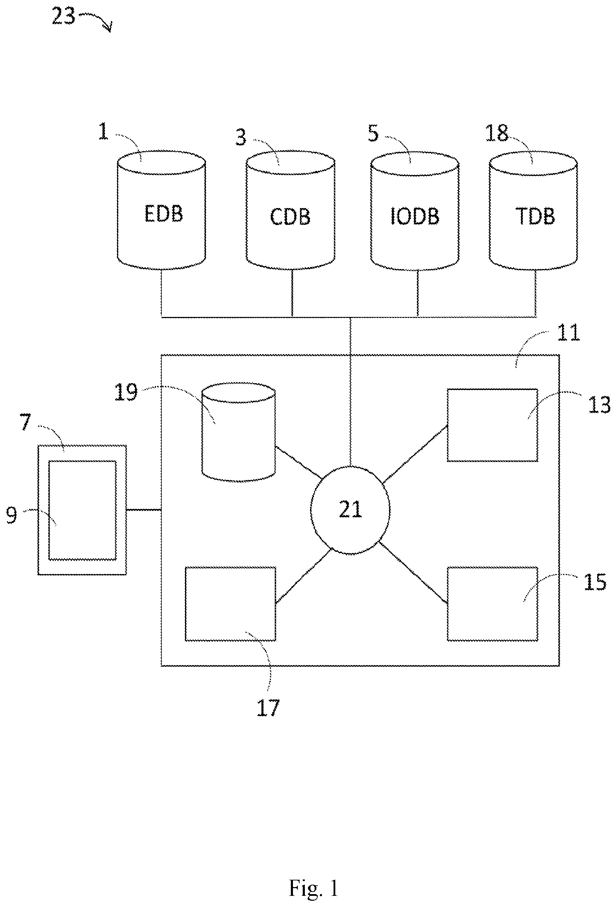

Turning to **FIG. 1****,** the computer implemented exposomic system **23** may comprise one or more of the following: (a) an exposome biochemical signatures database (EDB) **1,** the EDB may further comprise biochemical signature information for a plurality of subjects; (b) a clinical database (CBD) **3,** the CBD may further comprise clinical phenotype information for a plurality of subjects; (c) an intervention requirement database (IODB) 5, the IODB may further comprise information on intervention outcome information for at least one phase of at least one intervention; (d) a treatment database (TDB) **18;** and (e) a computer system **11** that may comprise a processing unit (CPU, also "processor" and "computer processor" herein) **21,** which can be a single core or a multi core processor, or a plurality of processor for parallel processing. The processor **21** may execute a sequence of machine-readable instructions embodied in a program or software, for example, (i) an association software module located on storage unit **19** i.e., memory, communicatively coupled to the EDB **1** and the CDB 3, the association software module may be programmed to determine an association between the exposomic features and the clinical phenotype information for at least one of the plurality of subjects, and (ii) a recommendation software module located on memory **19.** The software may be loaded from the memory **19** into random access memory (RAM) **17** or read-only memory (ROM) **17** that may provide an intervention recommendation for the at least one of the plurality of subjects based at least in part on the exposomic features, the clinical phenotype information, the intervention outcome information, and the association between the exposomic features and the clinical phenotype information for the at least one of the plurality of subjects.

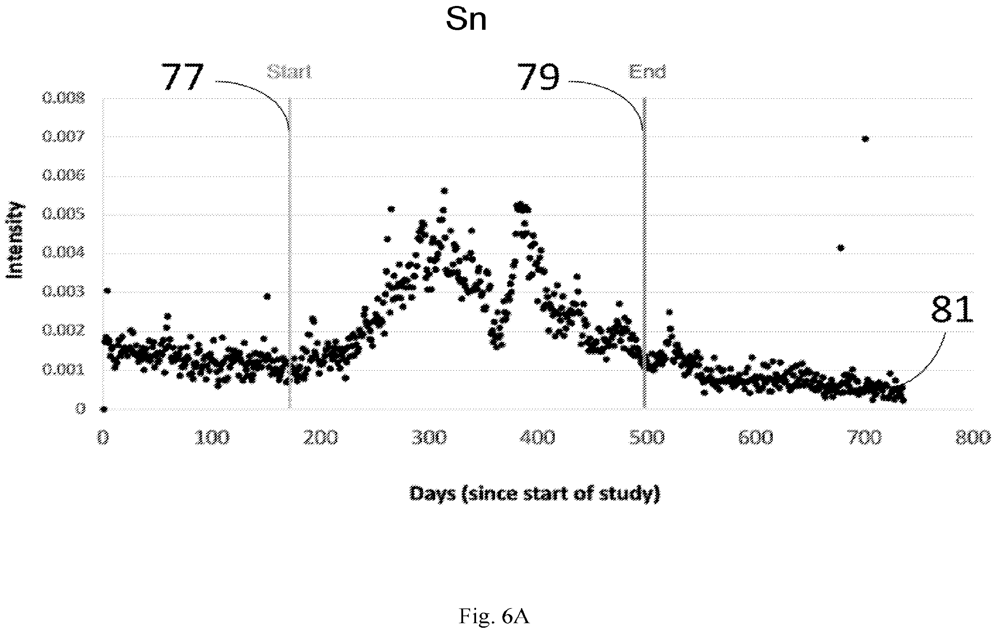

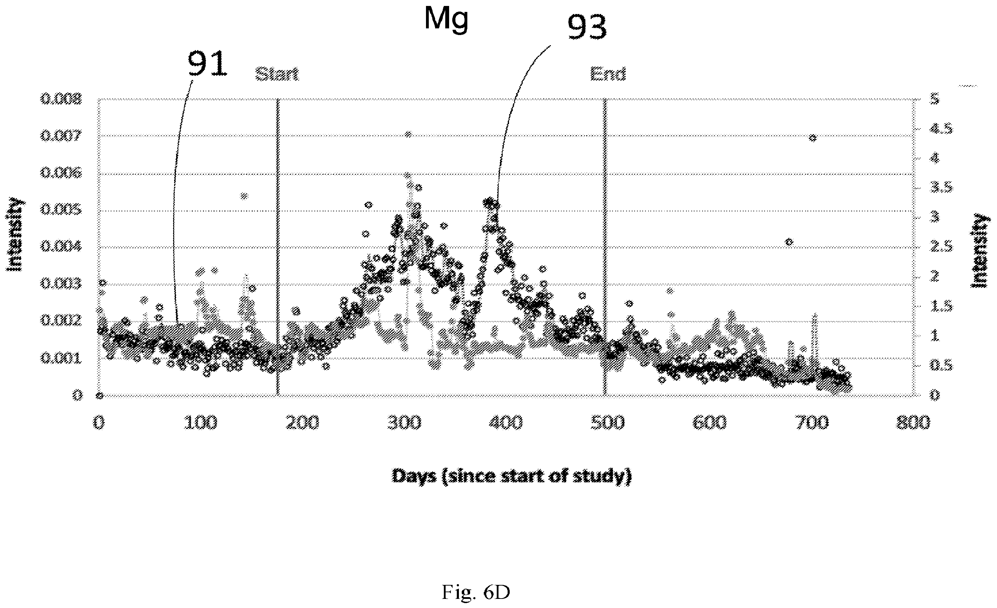

In some embodiments, the exposome biochemical signatures database (EDB) 1 may comprise exposomic features from a plurality of subjects. Exposome biochemical signatures may comprise biochemical signatures of perfluoro compounds, parabens, phthalates, lipids, amino acids, metabolites, peptides, metals, derivatives thereof, or any combination thereof, as seen in **FIG. 11****.** In some embodiments, exposome biochemical signatures are analyzed or acquired as a function of a subjects' lives (e.g., as a function of aging or as a function of time), in this case, the collection of exposome biochemical signatures, as seen in **FIG. 6A-6D****,** may be analyzed to produce one or more exposomic features,. In some embodiments, the period of time represented by an exposome biochemical signature may comprise at least 1 hour, at least 1 day, at least 1 week, at least 1 month, at least 1 year, or any combination thereof.

In some embodiments, the number of exposome biochemical signatures may comprise about 10 signatures to about 100,000 signatures. In some embodiments, the number of exposome biochemical signatures may comprise about 10 signatures to about 100 signatures, about 10 signatures to about 500 signatures, about 10 signatures to about 1,000 signatures, about 10 signatures to about 5,000 signatures, about 10 signatures to about 7,000 signatures, about 10 signatures to about 10,000 signatures, about 10 signatures to about 20,000 signatures, about 10 signatures to about 50,000 signatures, about 10 signatures to about 100,000 signatures, about 100 signatures to about 500 signatures, about 100 signatures to about 1,000 signatures, about 100 signatures to about 5,000 signatures, about 100 signatures to about 7,000 signatures, about 100 signatures to about 10,000 signatures, about 100 signatures to about 20,000 signatures, about 100 signatures to about 50,000 signatures, about 100 signatures to about 100,000 signatures, about 500 signatures to about 1,000 signatures, about 500 signatures to about 5,000 signatures, about 500 signatures to about 7,000 signatures, about 500 signatures to about 10,000 signatures, about 500 signatures to about 20,000 signatures, about 500 signatures to about 50,000 signatures, about 500 signatures to about 100,000 signatures, about 1,000 signatures to about 5,000 signatures, about 1,000 signatures to about 7,000 signatures, about 1,000 signatures to about 10,000 signatures, about 1,000 signatures to about 20,000 signatures, about 1,000 signatures to about 50,000 signatures, about 1,000 signatures to about 100,000 signatures, about 5,000 signatures to about 7,000 signatures, about 5,000 signatures to about 10,000 signatures, about 5,000 signatures to about 20,000 signatures, about 5,000 signatures to about 50,000 signatures, about 5,000 signatures to about 100,000 signatures, about 7,000 signatures to about 10,000 signatures, about 7,000 signatures to about 20,000 signatures, about 7,000 signatures to about 50,000 signatures, about 7,000 signatures to about 100,000 signatures, about 10,000 signatures to about 20,000 signatures, about 10,000 signatures to about 50,000 signatures, about 10,000 signatures to about 100,000 signatures, about 20,000 signatures to about 50,000 signatures, about 20,000 signatures to about 100,000 signatures, or about 50,000 signatures to about 100,000 signatures. In some embodiments, the number of exposome biochemical signatures may comprise about 10 signatures, about 100 signatures, about 500 signatures, about 1,000 signatures, about 5,000 signatures, about 7,000 signatures, about 10,000 signatures, about 20,000 signatures, about 50,000 signatures, or about 100,000 signatures. In some embodiments, the number of exposome biochemical signatures may comprise at least about 10 signatures, about 100 signatures, about 500 signatures, about 1,000 signatures, about 5,000 signatures, about 7,000 signatures, about 10,000 signatures, about 20,000 signatures, or about 50,000 signatures. In some embodiments, the number of exposome biochemical signatures may comprise at most about 100 signatures, about 500 signatures, about 1,000 signatures, about 5,000 signatures, about 7,000 signatures, about 10,000 signatures, about 20,000 signatures, about 50,000 signatures, or about 100,000 signatures.

In some embodiments, the exposome biochemical signatures may be obtained by assaying biological samples of a plurality of subjects. In some embodiments, the biological samples may comprise a tooth, nail or hair shaft sample. In some embodiments, the exposome biochemical signatures may be obtained using laser ablation-inductively coupled plasma mass spectrometry measurements, laser induced breakdown spectroscopy measurements, mass spectrometry measurements, Raman spectroscopy measurements, immunohistochemistry measurements, molecular tagging (with a fluorophore, for example), nuclear magnetic resonance, chromatography, or any combination thereof. In some embodiments, laser ablation-inductively coupled plasma mass spectrometry measurements may measure one or more element chemicals. In some embodiments, the one or more element chemicals comprise aluminum, arsenic, barium, bismuth, calcium, copper, iodide, lead, lithium, magnesium, manganese, phosphorus, sulfur, tin, strontium, zinc, or any combination thereof chemicals as described elsewhere herein e.g., **Table 1** and/or **Table 2.** In some embodiments, the exposome biochemical signatures information may comprise exposome temporal biochemical responses of the plurality of subjects. In some embodiments, the biochemical information may comprise fluorescence images of the biological samples. In some embodiments, the exposome biochemical signatures may comprise spatial maps of Raman spectra of the biological samples of the plurality of subjects. In some embodiments, the exposomic features may be associated with a disease or disorder. In some embodiments, the disease or disorder comprises psychological, cardiac, gastroenterological, pulmonary, neurological, circulatory, nephrological, or any combination thereof disease or disorders. In some embodiments, the disease or disorder comprises cancer, e.g., pediatric cancer, lung cancer, etc. In some embodiments the disease or disorder comprises, for example, autism spectrum disorder (ASD), attention deficit hyperactivity disorder (ADHD), amyotrophic lateral sclerosis (ALS), schizophrenia, irritable bowel disease (IBD), pediatric kidney disease, kidney transplant rejection, pediatric cancer or any combination thereof.

In some embodiments, the plurality of chemicals is selected from the chemicals listed in **Table 1.** In some embodiments, the plurality of chemicals includes at least 50%, 60%, 70%, 80% or 90% of the isotopes included in **Table 1.**

**Table 1. List of Chemicals**

| Chemicals | Element Name |
|---|---|
| Li-7 (Li) | lithium |
| Mg-24 (Mg) | magnesium |
| Mg-25 (Mg25) | magnesium |
| Al-27 (Al) | aluminum |
| P-31 (P) | phosphorus |
| S-34 (S) | sulfur |
| Ca-44 (Ca) | calcium |
| Ca-43 (Ca43) | calcium |
| Cr-52 (Cr) | chromium |
| Mn-55 (Mn) | manganese |
| Fe-56 (Fe) | iron |
| Co-59 (Co) | cobalt |
| Ni-60 (Ni) | nickel |
| Cu-63 (Cu) | copper |
| Zn-66 (Zn) | zinc |
| As-75 (As) | arsenic |
| Sr-88 (Sr) | strontium |
| Cd-111 (Cd) | cadmium |
| Sn-118 (Sn) | tin |
| I-127 (I) | iodine |
| Ba-138 (Ba) | barium |
| Hg-201 (Hg) | mercury |
| Pb-208 (Pb) | lead |
| Bi-209 (Bi) | bismuth |
| Mo-95(Mo) | molybdenum |

In some embodiments, the plurality of chemicals is selected from the chemicals listed in **Table 2. In** some embodiments, the plurality of chemicals includes at least 50%, 60%, 70%, 80% or 90% of the isotopes included in Table 2.

**Table 2. List of Chemicals**

| Chemicals | Element Name |
|---|---|
| Li7 | lithium |
| Mg24 | magnesium |
| A127 | aluminum |
| P31 | phosphorus |
| S34 | sulfur |
| Ca44 | calcium |
| V51 | vanadium |
| Cr52 | chromium |
| Mn55 | manganese |
| Fe56, Fe57 | iron |
| Co59 | cobalt |
| Ni60 | nickel |
| Cu63 | copper |
| Zn66 | zinc |
| As75 | arsenic |
| Sr88 | strontium |
| Cd111 | cadmium |
| Sn118 | tin |
| Sb121 | antimony |
| I127 | iodine |
| Ba138 | barium |
| Hg201 | mercury |
| Pb208 | lead |
| Bi209 | bismuth |

In some embodiments, one or more exposomic features are calculated from one or more dynamic exposomic biochemical signatures. Exposomic features derived through data analysis may comprise descriptive statistics or parameters which are utilized in subsequent statistical, machine learning, or artificial intelligence models. Such exposomic features may comprise standard descriptive metrics such as the mean, median, mode, and range, and/or associated measures of error and/or variation such as standard deviation, variance, confidence intervals, and/or related metrics of the one or more dynamic exposomic signatures. The derivation of exposomic features may comprise the application of computational methods to derive linear slope, non-linear parameters describing curvature of the one or more dynamic exposomic signatures, abrupt changes in intensity of the one or more dynamic exposomic signatures, changes in baseline intensity of the one or more dynamic exposomic signatures, changes of the frequency-domain representation of the one or more dynamic exposomic signatures, changes of the power-spectral domain representation of the one or more dynamic exposomic signatures, recurrence quantification analysis parameters, cross-recurrence quantification analysis parameters, joint recurrence quantification analysis parameters, multidimensional recurrence quantification analysis parameters, estimation of the lypanuv spectra or maximum Lyapunov exponent or any combination thereof.

In some embodiments, the one or more exposomic features comprise a measurement of temporal dynamics of the one or more dynamic exposomic signatures. In some embodiments, the measurement of the temporal dynamics comprise: linear slope, non-linear parameters describing curvature of the one or more dynamic exposomic signatures, abrupt changes in intensity of the one or more dynamic exposomic signatures, changes in baseline intensity of the one or more dynamic exposomic signatures, changes of the frequency-domain representation of the one or more dynamic exposomic signatures, changes of the power-spectral domain representation of the one or more dynamic exposomic signatures, recurrence quantification analysis parameters, cross-recurrence quantification analysis parameters, joint recurrence quantification analysis parameters, multidimensional recurrence quantification analysis parameters, estimation of the lypanuv spectra, maximum Lyapunov exponent, or any combination thereof.

In some embodiments, recurrence quantification analysis, cross-recurrence quantification analysis, joint recurrence quantification analysis, multi-dimensional recurrence quantification analysis of the one or more exposomic biochemical signatures may be used to derive descriptive statistics and/or parameters that are utilized in to train predictive models, described elsewhere herein.

In some instances, the recurrence quantification analysis parameters may comprise recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, and number of the most probable recurrences.

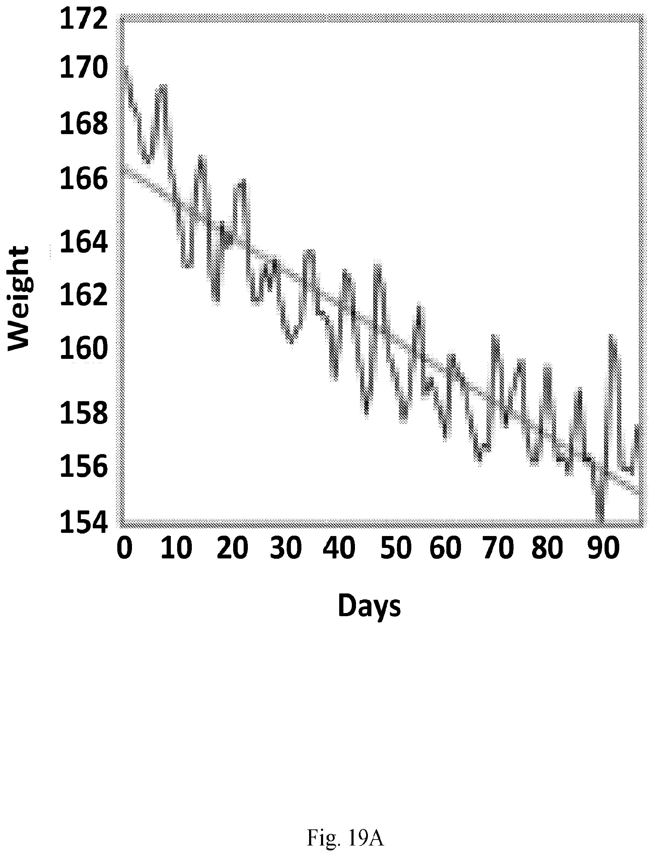

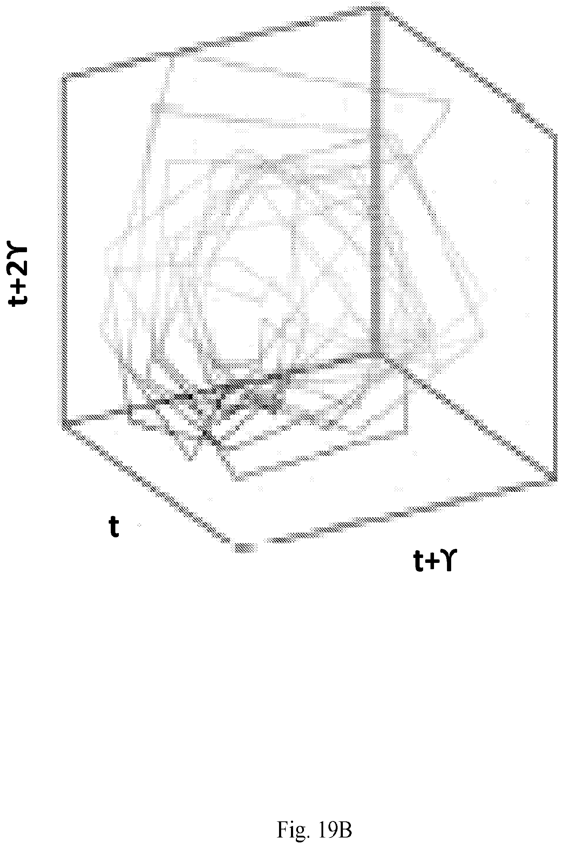

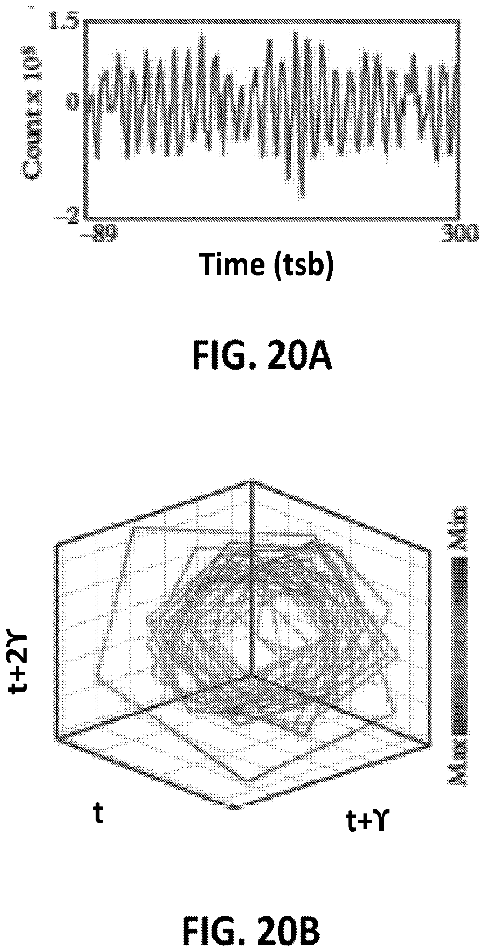

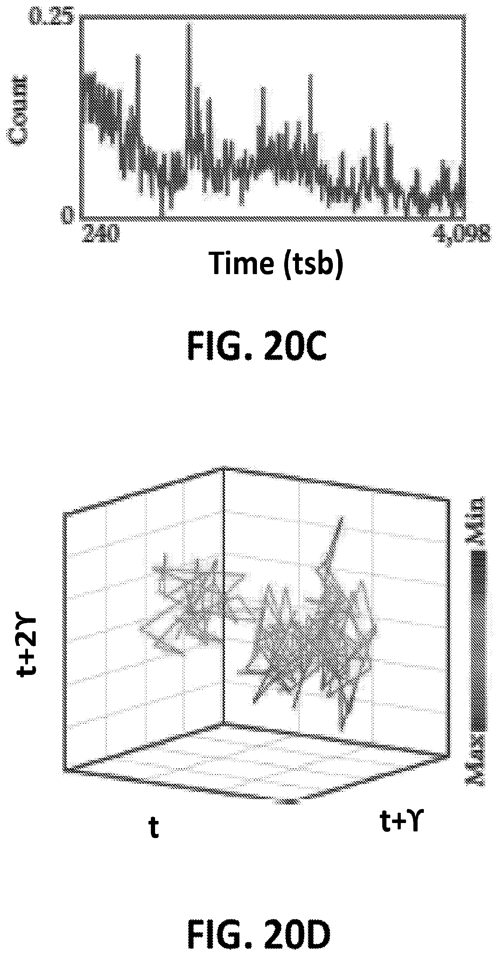

In some instances, the one or more exposomic features may be derived from one or more attractors **(****FIGS. 19B****,** **20B****,** **20D****),** whereby the one or more attractors are generated from the one or more dynamic exposomic biochemical signatures **(****FIG. 19A****,** **20A****,** **20C****).** In some embodiments, the one or more attractors are analyzed by potential energy analysis thereby producing a potential energy data space.

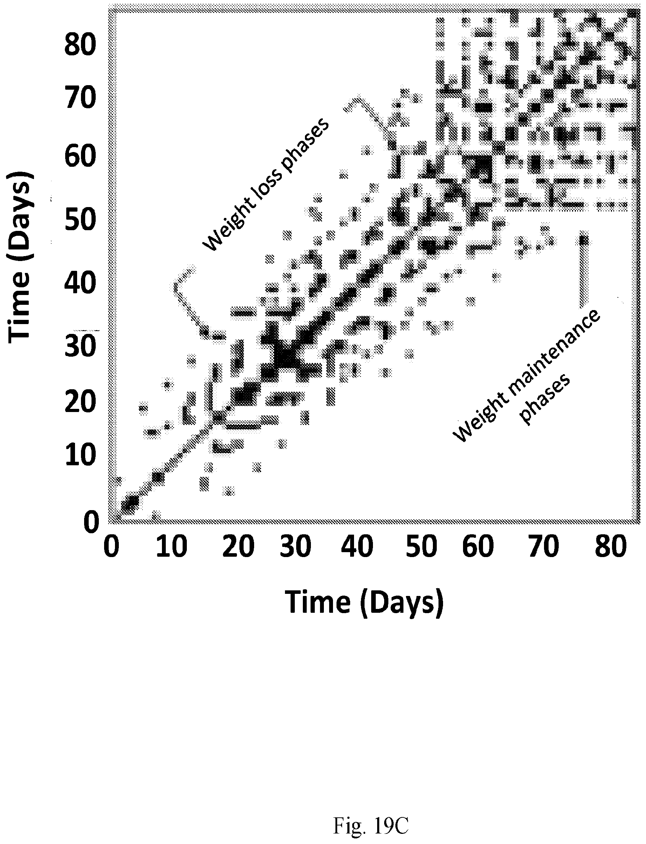

In some embodiments, a dynamic relationship **(****FIG. 19C****)** is established between the one or more attractors' signal recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, or any combination thereof, may be analyzed and provided as a feature. In some embodiments, the dynamic relationship is determined by cross-convergent mapping (CCM).

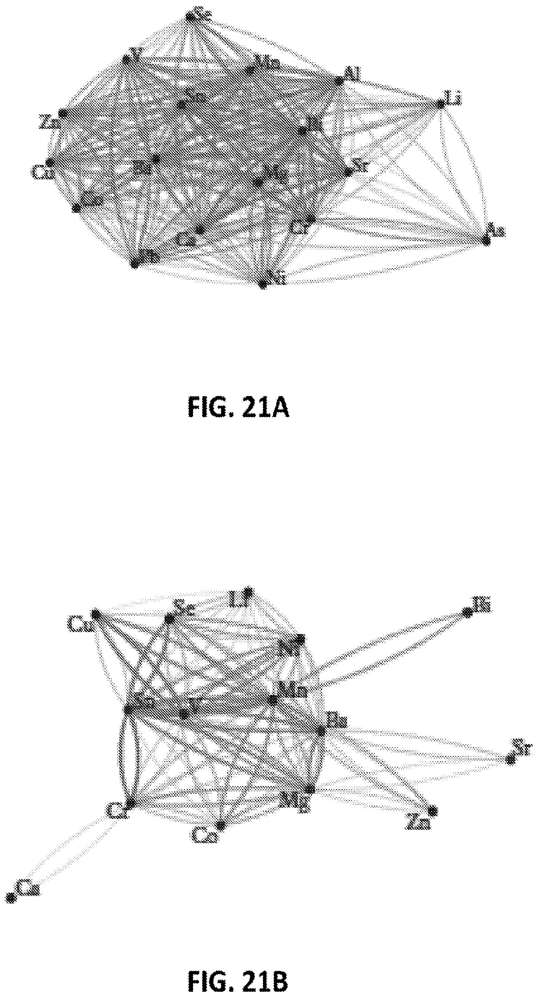

In some instances, a network may be constructed **(****FIGS. 21A-B)** of the one or more attractors based on similarity of the one or more attractors' temporal exposomic data signal recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, or any combination thereof. In some embodiments, one or more exposomic features of the network of the one or more attractors is analyzed to determine network connectivity, efficiency, feature importance, pathway importance, related graph-theory based metrics, or any combination thereof analysis.

In some embodiments, the one or more exposomic features of the one or more dynamic exposomic signatures comprise phenotypic exposomic features. The phenotypic exposomic features may comprise: electrocardiogram (ECG), electroencephalography, magnetic resonance imaging (MRI), functional magnetic resonance imaging (fMRI), positron emission tomography (PET), genomic, epigenomic, transcriptomic, proteomic, metabolomic, or any combination thereof data.

In some embodiments, the phenotypic exposomic features comprise molecular phenotypes. In some instances, the molecular phenotypes are determined by unsupervised analysis, where the unsupervised analysis comprises clustering, dimensionality-reduction, factor analysis, stacked autoencoding, or any combination thereof.

In some embodiments, the CBD **3** may comprise clinical phenotype data for subjects. In some embodiments, clinical phenotype data comprise clinical metadata for a plurality of subjects. In some embodiments, the clinical metadata may comprise the subject's age, gender, weight, height, blood type, eye vision, current diseases, past history of family diseases, or any combination thereof. In some embodiments, the clinical metadata and exposome biochemical signature of a subject may be considered independent or in combination to determine if a subject would be a suitable candidate for an intervention.

In some embodiments, the IODB **5** may comprise intervention outcome information. In some embodiments, the intervention outcome information may comprise eligibility criterion for one or more intervention. In some embodiments, the intervention may comprise a phase 1, 2, 3 or any combination thereof intervention. In some embodiments, the intervention outcome information may comprise information for one or more subjects' intervention outcome classification comprising: positive responder, negative responder, or non-responder.

In some embodiments, the TDB **18,** may comprise exposomic features that pertain at least in part to pharmaceutical and nutraceutical treatments. In some embodiments, the exposomic features of pharmaceutical and nutraceutical may comprise one or more reference exposomic features of subjects without disease or disorders, one or more pre-treatment exposomic features of subjects with a disease or disorder, and one or more post-treatment exposomic features of subjects with a disease or disorder obtained by assaying one or more biological sample of one or more subjects.

In some embodiments, the analysis of the differences between the one or more pre-treatment exposomic features and the one or more post-treatment exposomic features to the one or more reference dynamic profile biochemical signatures may be used to determine one or more optimal pharmaceutical, nutraceutical, or any combination thereof treatments for a subject with a disease or disorder. In some embodiments, a difference of the one or more post-treatment exposomic features towards the one or more reference exposomic features may provide the basis for recommending one or more pharmaceutical or nutraceuticals for a subject with a disease or disorder and may be used in combination with one or more subjects' exposome biochemical signature to recommend optimal treatments to prevent or treat one or more diseases or disorders to the one or more subjects. In some embodiments, the disease or disorder comprises psychological, cardiac, gastroenterological, pulmonary, neurological, circulatory, nephrological, or any combination thereof disease or disorders. In some embodiments, the disease or disorder comprises cancer, e.g., pediatric cancer, lung cancer, etc. In some embodiments the disease or disorder comprises, for example, autism spectrum disorder (ASD), attention deficit hyperactivity disorder (ADHD), amyotrophic lateral sclerosis (ALS), schizophrenia, irritable bowel disease (IBD), pediatric kidney disease, kidney transplant rejection, pediatric cancer or any combination thereof. In some embodiments, the criterion for an optimal pharmaceutical or nutraceutical treatment may comprise a complement to the deficiencies of a given subject's exposome biochemical signatures of the one or more subjects.

In some embodiments, the difference of one or more features of the post-treatment exposomic features to the one or more reference exposomic features. In some embodiment, the feature comprises an overall mean, a measure of variability, a moving average, etc., or any combination thereof features, as described elsewhere herein.

In some embodiments, the difference of the features comprises a difference by about 10 % to about 100 %. In some embodiments, the difference of the features comprises a difference by about 10 % to about 20 %, about 10 % to about 30 %, about 10 % to about 40 %, about 10 % to about 50 %, about 10 % to about 60 %, about 10 % to about 70 %, about 10 % to about 80 %, about 10 % to about 90 %, about 10 % to about 100 %, about 20 % to about 30 %, about 20 % to about 40 %, about 20 % to about 50 %, about 20 % to about 60 %, about 20 % to about 70 %, about 20 % to about 80 %, about 20 % to about 90 %, about 20 % to about 100 %, about 30 % to about 40 %, about 30 % to about 50 %, about 30 % to about 60 %, about 30 % to about 70 %, about 30 % to about 80 %, about 30 % to about 90 %, about 30 % to about 100 %, about 40 % to about 50 %, about 40 % to about 60 %, about 40 % to about 70 %, about 40 % to about 80 %, about 40 % to about 90 %, about 40 % to about 100 %, about 50 % to about 60 %, about 50 % to about 70 %, about 50 % to about 80 %, about 50 % to about 90 %, about 50 % to about 100 %, about 60 % to about 70 %, about 60 % to about 80 %, about 60 % to about 90 %, about 60 % to about 100 %, about 70 % to about 80 %, about 70 % to about 90 %, about 70 % to about 100 %, about 80 % to about 90 %, about 80 % to about 100 %, or about 90 % to about 100 %. In some embodiments, the difference of the features may comprise a difference by about 10 %, about 20 %, about 30 %, about 40 %, about 50 %, about 60 %, about 70 %, about 80 %, about 90 %, or about 100 %. In some embodiments, the difference of the features may comprise a difference by at least about 10 %, about 20 %, about 30 %, about 40 %, about 50 %, about 60 %, about 70 %, about 80 %, or about 90%. In some embodiments, the difference of the features may comprise a difference by at most about 20 %, about 30 %, about 40 %, about 50 %, about 60 %, about 70 %, about 80 %, about 90 %, or about 100 %.

The computer system may further comprise a communication interface **13** (e.g., network adapter) for communicating with one or more other systems, and peripheral devices **15,** such as cache, other memory, data storage and/or electronic display adapters. The memory **17,** storage unit **19,** interface **13** and peripheral devices **15** are in communication with the CPU **21** through a communication bus (solid lines), such as a motherboard. The storage unit **19** can be a data storage unit (or data repository) for storing data. The computer system **11** can be operatively coupled to a computer network ("network") with the aid of the communication interface **13.** The network can be the Internet, an extranet, and/or an intranet that is in communication with the Internet. The network, in some embodiments, is a telecommunication and/or data network. The network can include one or more computer servers, which can enable distributed computing, such as cloud computing. The network, in some cases with the aid of the computer system **11,** can implement a peer-to-peer network, which may enable devices coupled to the computer system **11** to behave as a client or a server.

In some embodiment, the EDB **1,** CDB **3,** IODB **5,** and TDB **18** may be located on the network and accessed remotely by the computer system **11.** In some embodiments, the EDB **1,** CDB **3,** IODB **5,** and TDB **18** may reside on a secure encrypted network server that protects personal health information. In some embodiments, the EDB **1,** CDB **3,** IODB **5,** and TDB **18,** may be accessed remotely by one or more computer systems **11** within or external to a network of hospitals. In some embodiments, the EDB **1,** CDB **3,** IODB **5,** and TDB **18** and may be accessed by one or more subjects over secure network protocols to view recommendations and their personalized data.

The CPU **21** can execute a sequence of machine-readable instructions, which can be embodied in a program or software. The instructions may be stored in a memory location, such as the random-access memory **17.** The instructions can be directed to the CPU **21,** which can subsequently program or otherwise configure the CPU **21** to implement methods of the present disclosure. Examples of operations performed by the CPU **21** can include fetch, decode, execute, and writeback.

The CPU **21** can be part of a circuit, such as an integrated circuit. One or more other components of the system **11** can be included in the circuit. In some embodiments, the circuit is an application specific integrated circuit (ASIC).

The storage unit **19** can store files, such as drivers, libraries and saved programs. The storage unit **19** can store user data, e.g., user preferences and user programs. The computer system **11** in some cases can include one or more additional data storage units that are external to the computer system **11,** such as located on a remote server that is in communication with the computer system **11** through an intranet or the Internet.

The computer system **11** may communicate with one or more remote computer systems through the network. For instance, the computer system **11** can communicate with a remote computer system of a user (e.g., a health care provider, subjects, etc.). Examples of remote computer systems include personal computers (e.g., portable PC), slate or tablet PC's (e.g., Apple^{®} iPad, Samsung^{®} Galaxy Tab), telephones, Smart phones (e.g., Apples iPhone, Android-enabled device, Blackberry^{®}), or personal digital assistants. The user can access the computer system **11** via the network.

Methods as described herein can be implemented by way of machine (e.g., computer processor) executable code stored on an electronic storage location of the computer system **11,** such as, for example, on the memory **17** or electronic storage unit **19.** The machine executable or machine-readable code can be provided in the form of software. During use, the code can be executed by the processor **21.** In some embodiments, the code is retrieved from the storage unit **19** and stored on the random-access memory **17** for ready access by the processor **21.** In some situations, the storage unit **19** can be precluded, and machine-executable instructions are stored on the random-access memory **17.**

The code can be pre-compiled and configured for use with a machine having a processer adapted to execute the code or can be compiled during runtime. The code can be supplied in a programming language that can be selected to enable the code to execute in a pre-compiled or as-compiled fashion.

Aspects of the systems and methods provided herein, such as the computer system **11,** can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, may be used to implement the databases, etc. shown in the drawings. Volatile storage media include dynamic memory, such as main memory of such a computer platform. Tangible transmission media include coaxial cables; copper wire; and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **11** can include or be in communication with an electronic display **7** that comprises a user interface (UI) **9** for providing, for example, fluorescence image data, fluorescence intensity data, temporal profiles of inflammation, and machine learning classifications. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit **21,** described elsewhere herein.

### Predictive Models.

In another aspect, disclosed herein, but not claimed are trained predictive models implemented on the computer implemented exposomic system **23.** In some embodiments, the trained predictive models may be configured to provide retrospective or prospective predictions of subjects' probability of success for interventions, pharmaceutical or nutraceutical treatments for subjects in need thereof, or any combination thereof. In some embodiments, the trained predictive models may comprise a statistical, machine learning, artificial intelligence classifiers, an ensemble of classifiers, or any combination thereof.

The classifier may comprise one or more statistical, machine learning, or artificial intelligence algorithms. Examples of utilized algorithms may include a support vector machine (SVM), a naive Bayes classification, a random forest, a neural network (such as a deep neural network (DNN), a recurrent neural network (RNN), a deep RNN, a long short-term memory (LSTM) recurrent neural network (RNN), or a gated recurrent unit (GRU), or other supervised learning algorithm or unsupervised machine learning, statistical, deep-learning algorithm, shallow-learning algorithm for classification and regression. The classifier may likewise involve the estimation of ensemble models, comprised of multiple predictive models, and utilize techniques such as gradient boosting, for example in the construction of gradient-boosting decision trees. The classifier may be trained using one or more training datasets corresponding to patient data. In some embodiments, the one or more training datasets may comprise exposome biochemical signatures, dynamic exposome biochemical signatures, clinical metadata, clinical trial information, exposomic features of pharmaceutical and nutraceutical treatments, or any combination thereof.

In some embodiments, training data features may comprise subjects' dynamic exposomic biochemical signature data generated from biological samples. For each biological sample of a given subject, a plurality of positions of a reference line on a biological sample of the training subject may be sampled in order to generate measurements therefrom, thereby obtaining a plurality of exposomic biochemical signatures. Each exposomic biochemical signature in the corresponding plurality of exposomic biochemical signatures corresponds to a different position in the corresponding plurality of positions, and each position in the corresponding plurality of positions represents a different period of growth of the corresponding biological sample. Next, each respective position of the biological sample is analyzed (e.g., using a laser ablation-inductively coupled-plasma mass spectrometer (LA-ICP-MS), a fluorescence image sensor, or a Raman spectrometer) to obtain a plurality of traces. Each trace in the corresponding plurality of traces corresponds to an abundance measurement of a corresponding substance, which are over time collectively determined from the corresponding plurality of dynamic exposome biochemical signatures.

In some embodiments, labels may comprise intervention outcomes such as, for example, a positive response, negative response (i.e., adverse response), or a non-responder. Intervention outcomes may comprise a temporal characteristic associated with the classification of a positive response, negative response, or non-responder event to the duration of time after administration of the treatment provided during intervention. Such a period of time may be, for example, about 1 hour, about 2 hours, about 3 hours, about 4 hours, about 6 hours, about 8 hours, about 10 hours, about 12 hours, about 14 hours, about 16 hours, about 18 hours, about 20 hours, about 22 hours, about 24 hours, about 2 days, about 3 days, about 4 days, about 5 days, about 6 days, about 7 days, about 10 days, about 2 weeks, about 3 weeks, about 4 weeks, about 1 month, about 2 months, about 3 months, about 4 months, about 6 months, about 8 months, about 10 months, about 1 year, or more than about 1 year.

Input features for training the classifier may be structured by aggregating the data into bins or alternatively using one-hot encoding. Inputs may also include feature values or vectors derived from the previously mentioned inputs, such as cross-correlations calculated between separate exposomic features or other measurements over a fixed period of time, and the discrete derivative or the finite difference between successive measurements, described elsewhere herein. Such a period of time may be, for example, about 1 hour, about 2 hours, about 3 hours, about 4 hours, about 6 hours, about 8 hours, about 10 hours, about 12 hours, about 14 hours, about 16 hours, about 18 hours, about 20 hours, about 22 hours, about 24 hours, about 2 days, about 3 days, about 4 days, about 5 days, about 6 days, about 7 days, about 10 days, about 2 weeks, about 3 weeks, about 4 weeks, about 1 month, about 2 months, about 3 months, about 4 months, about 6 months, about 8 months, about 10 months, about 1 year, or more than about 1 year.

Training records may be constructed from sequences of observations. Such sequences may comprise a fixed length for ease of data processing. For example, sequences may be zero-padded or selected as independent subsets of a single patient's records
In order to train the classifier model (e.g., by determining weights and correlations of the model) to generate real-time classifications or predictions, the model can be trained using datasets. Such datasets may be sufficiently large to generate statistically significant classifications or predictions. For example, datasets may comprise: databases of de-identified data including dynamic profile biological signature data and other clinical metadata measurements from a hospital or other clinical setting.

Datasets may be split into subsets (e.g., discrete or overlapping), such as a training dataset, a development dataset, and a test dataset. For example, a dataset may be split into a training dataset comprising 80% of the dataset and a test dataset comprising 20% of the dataset. The training dataset may comprise about 10%, about 20%, about 30%, about 40%, about 50%, about 60%, about 70%, about 80%, or about 90% of the dataset. The development dataset may comprise about 10%, about 20%, about 30%, about 40%, about 50%, about 60%, about 70%, about 80%, or about 90% of the dataset. The test dataset may comprise about 10%, about 20%, about 30%, about 40%, about 50%, about 60%, about 70%, about 80%, or about 90% of the dataset. Training sets (e.g., training datasets) may be selected by random sampling of a set of data corresponding to one or more subject cohorts to ensure independence of sampling. Alternatively, training sets (e.g., training datasets) may be selected by proportionate sampling of a set of data corresponding to one or more subject cohorts to ensure independence of sampling.

To improve the accuracy of model predictions and reduce overfitting of the model, the datasets may be augmented to increase the number of samples within the training set. For example, data augmentation may comprise rearranging the order of observations in a training record. To accommodate datasets having missing observations, methods to impute missing data may be used, such as forward-filling, back-filling, linear interpolation, and multi-task Gaussian processes. Datasets may be filtered to remove confounding factors. For example, within a database, a subset of subjects may be excluded.

In some embodiments, data science techniques, such as dropout or regularization, may be used during training the classifier to prevent overfitting. The neural network may comprise a plurality of sub-networks, each of which is configured to generate a classification or prediction of a different type of output information (e.g., which may be combined to form an overall output of the neural network). The classifier may alternatively utilize statistical or related algorithms including random forest, classification and regression trees, support vector machines, discriminant analyses, regression techniques, as well as ensemble and gradient-boosted variations thereof.

In some embodiments, the systems and methods of the present disclosure are deployed in a hospital setting for patients that are active receiving treatment for their disease or disorder. When the classifier generates a classification or a prediction for an optimal pharmaceutical or nutraceutical, a notification (e.g., alert or alarm) may be generated and transmitted to a health care provider, such as a physician, nurse, or other member of the patient's treating team within a hospital. Notifications may be transmitted via an automated phone call, a short message service (SMS) or multimedia message service (MMS) message, an e-mail, an alert within a dashboard, or any combination thereof. The notification may comprise output information such as a prediction for the outcome of an intervention or an optimal pharmaceutical or nutraceutical.

To validate the performance of the classifier model, different performance metrics may be generated. For example, an area under the receiver-operating curve (AUROC) may be used to determine the predictive capability of the classifier. For example, the classifier may use classification thresholds which are adjustable, such that specificity and sensitivity are tunable, and the receiver-operating curve (ROC) can be used to identify the different operating points corresponding to different values of specificity and sensitivity.

In some embodiments, the performance of predictive methods are assessed by constructing tables to provide the frequency and overlap of predicted positive cases and actual positive cases, predicted positive cases and actual negative cases, predicted negative cases and actual negative cases, and/or predicted negative cases and actual positive cases. In the some instances, the tables constructed may be confusion matrices. In some cases, cross-tabulation of the confusion matrices may provide sensitivity, specificity, accuracy, and related performance metrics associated with systems and methods described elsewhere herein, at a given predictive threshold.

In some embodiments, such as when datasets are not sufficiently large, cross-validation may be performed to assess the robustness of a classifier model across different training and testing datasets.

To calculate performance metrics such as sensitivity, specificity, accuracy, positive predictive value (PPV), negative predictive value (NPV), AUPRC, AUROC, or similar, the following definitions may be used. A "false positive" may refer to an outcome in which a positive outcome or result has been incorrectly generated (e.g., a subject classified as a positive responder to an intervention, yet they experience an adverse or negative effect of participating in the intervention). A "true positive" may refer to an outcome in which positive outcome or result has been correctly generated (e.g., the subject is classified as a positive responder to an intervention and they experience a positive response). A "false negative" may refer to an outcome in which a negative outcome or result has been generated (e.g., the subject is classified as a negative responder where the subject after participating in the intervention is a non-responder or a positive responder). A "true negative" may refer to an outcome in which a negative outcome or result has been generated (e.g., the subject is classified as a negative responder and after participating in the intervention the subject responds adversely to the pharmaceutical treatment of the intervention).

In some embodiments, the classifier may be trained until certain pre-determined conditions for accuracy or performance are satisfied, such as having minimum desired values corresponding to predictive accuracy measures. For example, the predictive accuracy measure may correspond to correct prediction for an outcome of an intervention or an optimal pharmaceutical or nutraceutical recommendation and/or selection. Examples of diagnostic accuracy measures may include sensitivity, specificity, positive predictive value (PPV), negative predictive value (NPV), accuracy, area under the precision-recall curve (AUPRC), and area under the curve (AUC) of a Receiver Operating Characteristic (ROC) curve (AUROC) corresponding to the diagnostic accuracy of detecting or predicting a disease or disorder.

For example, such a pre-determined condition may be that the sensitivity of predicting the outcome of an intervention or an optimal pharmaceutical or nutraceutical recommendation and/or selection comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the specificity of predicting the outcome of an intervention or an optimal pharmaceutical or nutraceutical recommendation and/or selection comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the positive predictive value (PPV) of predicting the outcome of an intervention or an optimal pharmaceutical or nutraceutical recommendation and/or selection comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the negative predictive value (NPV) of outcome of an intervention or an optimal pharmaceutical or nutraceutical recommendation and/or selection comprises a value of, for example, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

As another example, such a pre-determined condition may be that the area under the curve (AUC) of a Receiver Operating Characteristic (ROC) curve (AUROC) of predicting the outcome of an intervention or an optimal pharmaceutical or nutraceutical recommendation and/or selection comprises a value of at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

As another example, such a pre-determined condition may be that the area under the precision-recall curve (AUPRC) of predicting the outcome of an intervention or an optimal pharmaceutical or nutraceutical recommendation and/or selection comprises a value of at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

In some embodiments, the trained classifier may be trained or configured to predict the outcome of an intervention or an optimal pharmaceutical or nutraceutical recommendation and/or selection with a sensitivity of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

In some embodiments, the trained classifier may be trained or configured to predict the outcome of an intervention or an optimal pharmaceutical or nutraceutical recommendation and/or selection with a specificity of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

In some embodiments, the trained classifier may be trained or configured to predict the outcome of an intervention or an optimal pharmaceutical or nutraceutical recommendation and/or selection with a positive predictive value (PPV) of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

In some embodiments, the trained classifier may be trained or configured to predict the outcome of an intervention or an optimal pharmaceutical or nutraceutical recommendation and/or selection with a negative predictive value (NPV) of at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99%.

In some embodiments, the trained classifier may be trained or configured to predict the outcome of an intervention or an optimal pharmaceutical or nutraceutical recommendation and/or selection with an area under the curve (AUC) of a Receiver Operating Characteristic (ROC) curve (AUROC) of at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

In some embodiments, the trained classifier may be trained or configured to predict the outcome of an intervention or an optimal pharmaceutical or nutraceutical recommendation and/or selection with an area under the precision-recall curve (AUPRC) of at least about 0.10, at least about 0.15, at least about 0.20, at least about 0.25, at least about 0.30, at least about 0.35, at least about 0.40, at least about 0.45, at least about 0.50, at least about 0.55, at least about 0.60, at least about 0.65, at least about 0.70, at least about 0.75, at least about 0.80, at least about 0.85, at least about 0.90, at least about 0.95, at least about 0.96, at least about 0.97, at least about 0.98, or at least about 0.99.

In some embodiments, the classifier is a neural network or a convolutional neural network. See, Vincent et al., 2010, "Stacked denoising autoencoders: Learning useful representations in a deep network with a local denoising criterion," J Mach Learn Res 11, pp. 3371-3408; Larochelle et al., 2009, "Exploring strategies for training deep neural networks," J Mach Learn Res 10, pp. 1-40; and Hassoun, 1995, Fundamentals of Artificial Neural Networks, Massachusetts Institute of Technology.

SVMs are described in Cristianini and Shawe-Taylor, 2000, "An Introduction to Support Vector Machines," Cambridge University Press, Cambridge; Boser et al., 1992, "A training algorithm for optimal margin classifiers," in Proceedings of the 5th Annual ACM Workshop on Computational Learning Theory, ACM Press, Pittsburgh, Pa., pp. 142-152; Vapnik, 1998, Statistical Learning Theory, Wiley, New York; Mount, 2001, Bioinformatics: sequence and genome analysis, Cold Spring Harbor Laboratory Press, Cold Spring Harbor, N.Y.; Duda, Pattern Classification, Second Edition, 2001, John Wiley & Sons, Inc., pp. 259, 262-265; and Hastie, 2001, The Elements of Statistical Learning, Springer, New York; and Furey et al., 2000, Bioinformatics 16, 906-914,. When used for classification, SVMs separate a given set of binary labeled data with a hyper-plane that is maximally distant from the labeled data. For cases in which no linear separation is possible, SVMs can work in combination with the technique of 'kernels', which automatically realizes a non-linear mapping to a feature space. The hyper-plane found by the SVM in feature space corresponds to a non-linear decision boundary in the input space.

Decision trees are described generally by Duda, 2001, Pattern Classification, John Wiley & Sons, Inc., New York, pp. 395-396. Tree-based methods partition the feature space into a set of rectangles, and then fit a model (like a constant) in each one. In some embodiments, the decision tree is random forest regression. One specific algorithm that can be used is a classification and regression tree (CART). Other specific decision tree algorithms include, but are not limited to, ID3, C4.5, MART, and Random Forests. CART, ID3, and C4.5 are described in Duda, 2001, Pattern Classification, John Wiley & Sons, Inc., New York. pp. 396-408 and pp. 411-412. CART, MART, and C4.5 are described in Hastie et al., 2001, The Elements of Statistical Learning, Springer-Verlag, New York, Chapter 9. Random Forests are described in Breiman, 1999, "Random Forests-Random Features," Technical Report 567, Statistics Department, U.C. Berkeley, September 1999.

Clustering (e.g., unsupervised clustering model algorithms and supervised clustering model algorithms) is described at pages 211-256 of Duda and Hart, Pattern Classification and Scene Analysis, 1973, John Wiley & Sons, Inc., New York, (hereinafter "Duda 1973"). As described in Section 6.7 of Duda 1973, the clustering problem is described as one of finding natural groupings in a dataset. To identify natural groupings, two issues are addressed. First, a way to measure similarity (or dissimilarity) between two samples is determined. This metric (similarity measure) is used to ensure that the samples in one cluster are more like one another than they are to samples in other clusters. Second, a mechanism for partitioning the data into clusters using the similarity measure is determined. Similarity measures are discussed in Section 6.7 of Duda 1973, where it is stated that one way to begin a clustering investigation is to define a distance function and to compute the matrix of distances between all pairs of samples in the training set. If distance is a good measure of similarity, then the distance between reference entities in the same cluster will be significantly less than the distance between the reference entities in different clusters. However, as stated on page 215 of Duda 1973, clustering does not require the use of a distance metric. For example, a nonmetric similarity function s(x, x') can be used to compare two vectors x and x'. Conventionally, s(x, x') is a symmetric function whose value is large when x and x' are somehow "similar." An example of a nonmetric similarity function s(x, x') is provided on page 218 of Duda 1973. Once a method for measuring "similarity" or "dissimilarity" between points in a dataset has been selected, clustering requires a criterion function that measures the clustering quality of any partition of the data. Partitions of the data set that extremize the criterion function are used to cluster the data. See page 217 of Duda 1973. Criterion functions are discussed in Section 6.8 of Duda 1973. More recently, Duda et al., Pattern Classification, 2nd edition, John Wiley & Sons, Inc. New York, has been published. Pages 537-563 describe clustering in detail. More information on clustering techniques can be found in Kaufman and Rousseeuw, 1990, Finding Groups in Data: An Introduction to Cluster Analysis, Wiley, New York, N.Y.; Everitt, 1993, Cluster analysis (3d ed.), Wiley, New York, N.Y.; and Backer, 1995, Computer-Assisted Reasoning in Cluster Analysis, Prentice Hall, Upper Saddle River, New Jersey. Particular exemplary clustering techniques that can be used in the present disclosure include, but are not limited to, hierarchical clustering (agglomerative clustering using nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering, where no preconceived notion of what clusters should form when the training set is clustered, are imposed.

Regression models, such as that of the multi-category logit models, are described in Agresti, An Introduction to Categorical Data Analysis, 1996, John Wiley & Sons, Inc., New York, Chapter 8. In some embodiments, the classifier makes use of a regression model disclosed in Hastie et al., 2001, The Elements of Statistical Learning, Springer-Verlag, New York. In some embodiments, gradient-boosting models are used toward, for example, the classification algorithms described herein; these gradient-boosting models are described in Boehmke, Bradley; Greenwell, Brandon (2019). "Gradient Boosting". Hands-On Machine Learning with R. Chapman & Hall. pp. 221-245. ISBN 978-1-138-49568-5. In some embodiments, ensemble modeling techniques are used, for example, toward the classification algorithms described herein; these ensemble modeling techniques are described in the implementation of classification models herein, are described in Zhou Zhihua (2012). Ensemble Methods: Foundations and Algorithms. Chapman and Hall/CRC. ISBN 978-1-439-83003-1.

In some embodiments, the machine learning analysis is performed by a device executing one or more programs (e.g., one or more programs stored in the Non-Persistent Memory (i.e., RAM or ROM) 17 or in the storage unit **19** (i.e., hard-disk) in **FIG. 1** including instructions to perform the data analysis. In some embodiments, the data analysis is performed by a system comprising at least one processor (e.g., the processing core **21)** and memory (e.g., one or more programs stored in the Non-Persistent Memory **17** or in the storage unit **19)** comprising instructions to perform the data analysis

### Intervention Stratification Predictive Models

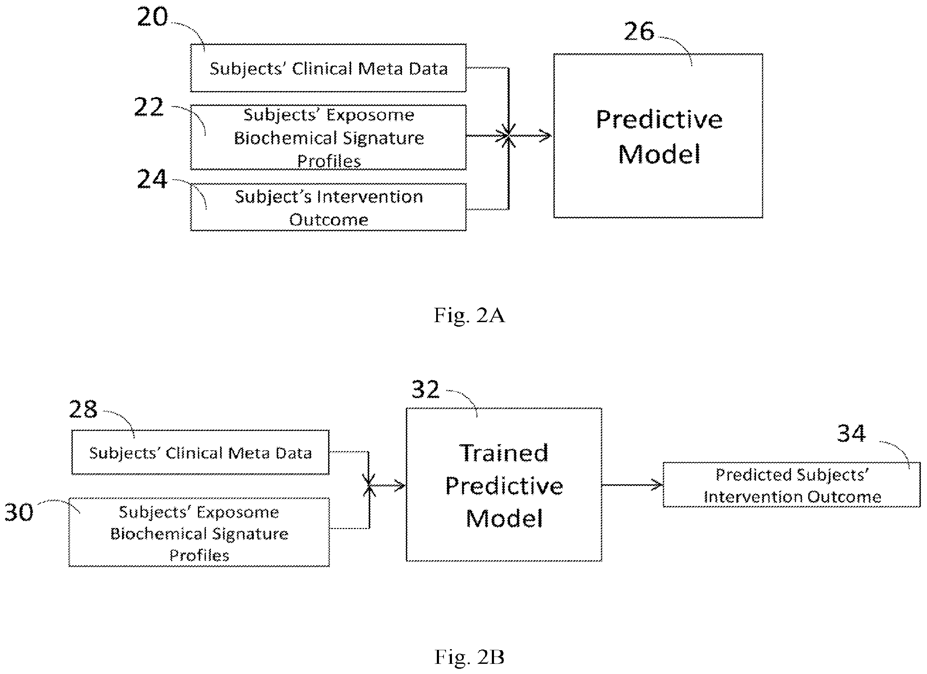

In some embodiments, the predictive model **26** may comprise one or more classifiers that may be trained to predict the probability of intervention outcomes for one or more subjects based on their exposomic features, as seen in **FIG. 2A****.** In some embodiments, the inputs for training the classifier may comprise subjects' clinical meta data **20,** subjects' exposomic biochemical signature data **22,** and the subjects' corresponding intervention outcome **24.** In some embodiments, the intervention outcome for a given subject may comprise, non-responder, adverse responder, or positive responder. In some embodiments, the predictive model may be initially trained on a dataset of one or more subjects having undergone treatment from the intervention. In some embodiments, training data sets used for training a classifier may be generated from, for example, the subjects' clinical meta data and the corresponding subjects' exposomic biochemical signature profiles, or features derived from the exposomic biochemical signature profiles, for example via RQA, described elsewhere herein, and intervention outcomes.

In some embodiments, clinical metadata features may comprise subjects' demographic information derived from electronic medical records (EMR),physiological measurements, and intervention outcomes. Additionally, training features may comprise clinical characteristics such as, for example, certain ranges or categories of dynamic exposome biochemical signature data. For example, a set of features collected from a given patient at a given time point may collectively serve as a signature of the CBD **3** and EDB **1,** which may be indicative of a health state or status of the subjects'.

In some embodiments, the trained predictive model **32** may comprise a trained classifier, described elsewhere herein, configured to provide predictions of the intervention outcome with regards to subjects interested in participating in an intervention, as seen in **FIG. 2B****.** In some embodiments, one or more subjects' clinical metadata **28** and corresponding exposomic features **30** may be fed as an input into the trained predictive model **32.** The trained predictive model **32** may then output a probability of a predicted subjects' trial outcome **34.** In some embodiments, the output probability of predicted subjects' trial outcome may comprise a classification of a positive responder, negative or adverse responder, or non-responder. In some embodiments, the subjects' clinical meta data may comprise clinical metadata e.g., subjects' age, gender, weight, height, blood type, eye vision, current diseases, past history of family diseases, or any combination thereof. Various machine learning techniques may be cascaded such that the output of a machine learning technique may also be used as input features to subsequent layers or subsections of the classifier.

### Optimal Pharmaceutical or Nutraceutical Selection Predictive Models

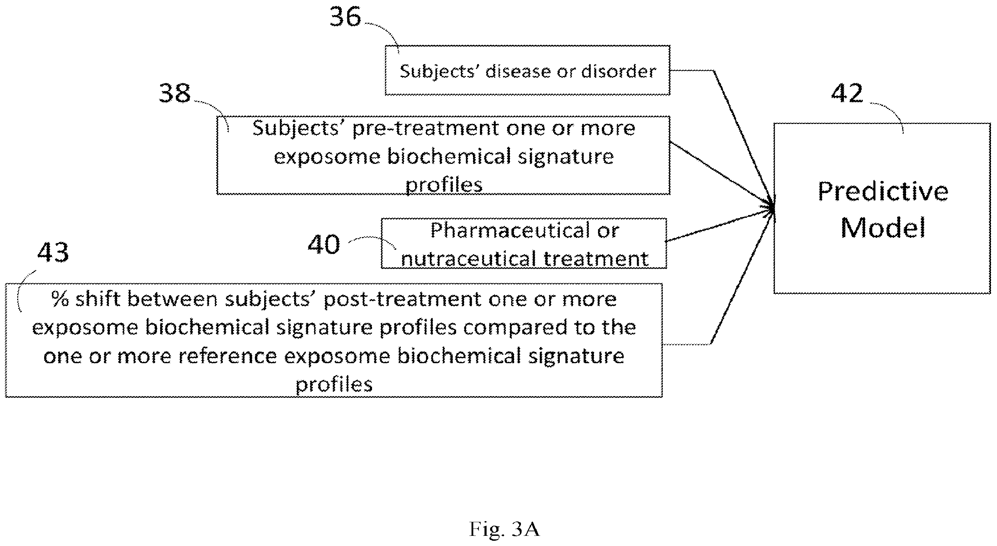

In some embodiments, the predictive model **42** may comprise one or more classifiers, described elsewhere herein, that may be trained to produce a trained predictive model **48** configured to predict the optimal pharmaceutical or nutraceutical to administered for a given disease or disorder for one or more subjects based on features derived from their respective one or more exposomic features, as seen in **FIG. 3A****,** described elsewhere herein. In some embodiments, the inputs for training statistical, machine learning, and/or artificial intelligence classifiers may comprise (a) subjects' disease or disorder **36;** (b) subjects' pre-treatment features derived from one or more exposomic features **38;** (c) the pharmaceutical or nutraceutical treatment administered **40;** and (d) the percent difference between the subjects' post-treatment features in one or more exposomic features compared to the one or more reference features derived from exposomic features of the one or more features derived from biochemical signatures of subjects without the disease or disorder.

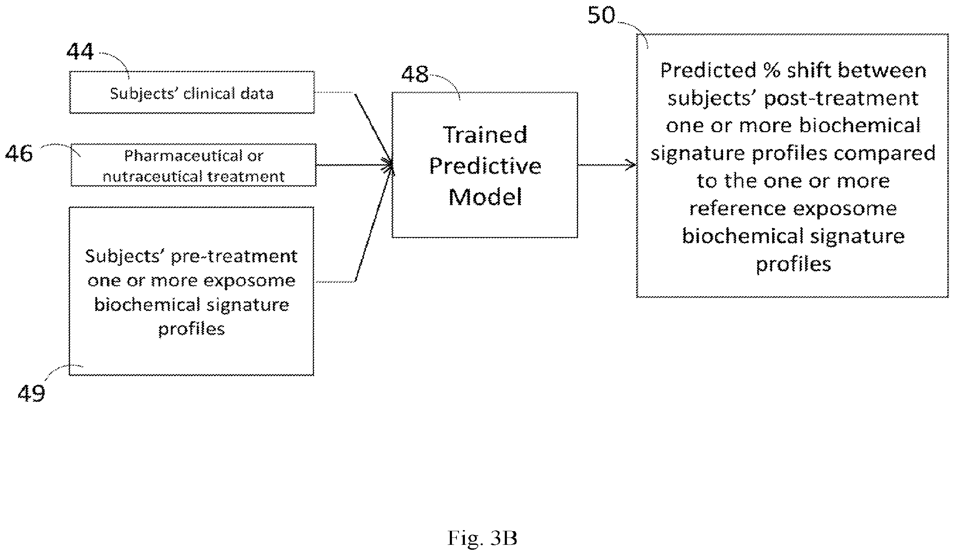

In some embodiments, the trained predictive model **48** may comprise a trained classifier, described elsewhere herein, configured to provide prediction **50** of the percent difference between subject's post-treatment one or more exposomic features and the one or more reference exposomic features of the one or, as seen in **FIG. 3B****.** In some embodiments, the trained predictive model may take as inputs: (a) the subjects' clinical data **44;** (b) the pharmaceutical or nutraceutical treatment under consideration **46;** and (c) the subjects' pre-treatment one or more exposomic features of. In some embodiments, one or more pharmaceutical or nutraceutical treatments may be considered.

### Exposome Cluster Analysis

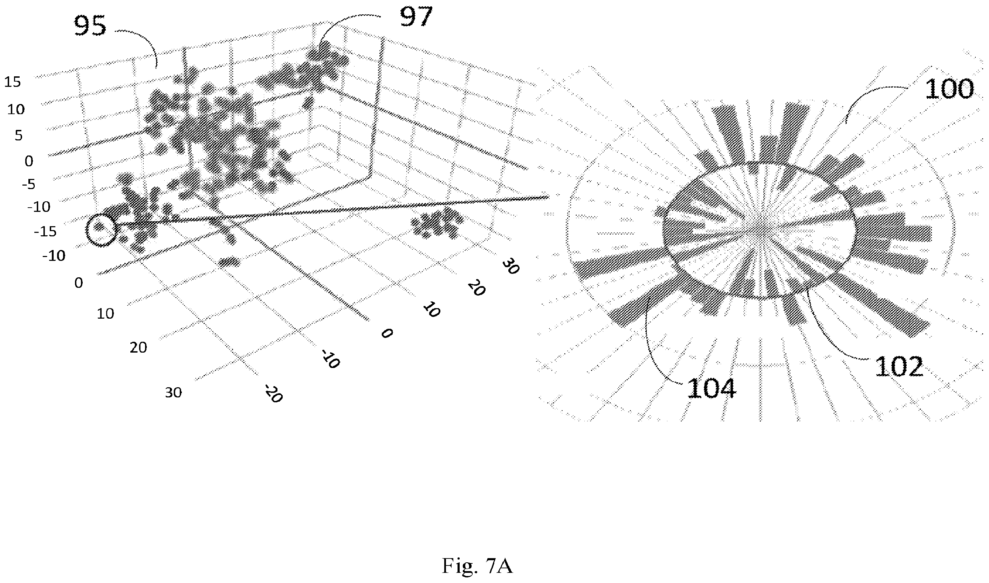

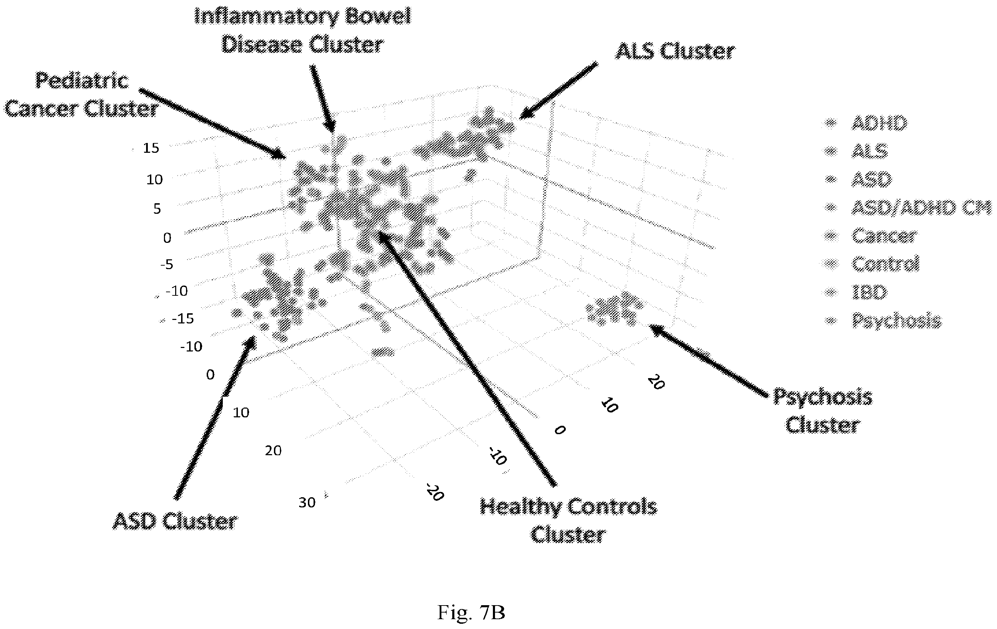

In some embodiments, one or more subjects' one or more exposome signature profiles may be analyzed by clustering methods to categorically classify or group subjects' based on disease or disorder as seen in **FIG. 7A-7B****.** One or more subjects' are represented by exposome data clusters **97, 100** of one or more exposomic features **104.** Subjects' one or more exposomic features may be compared to an average **102** of a cohort for analysis or classification. Alternatively, exposomic features may be used to sub-type subjects' prior to, during or after clinical intervention to understand which subjects may respond positively, negative, or have no response to a given intervention.

### Methods.

Aspects of the disclosure herein may comprise methods of intervention optimization and recommendation of optimal pharmaceutical and or nutraceutical recommendations for subjects suffering from a disease or disorder. In some embodiments, the methods described herein may be performed on the systems of the present disclosure described elsewhere herein.

Specifically, the invention relates to a method for evaluating the effects of an intervention in a plurality of subjects, the method comprising:
sampling, for each respective subject in the plurality of subjects, each respective position in a plurality of positions along a reference line on a corresponding biological sample associated with chemical dynamics of the respective subject, thereby obtaining a corresponding plurality of chemical samples for the respective subject, each chemical sample in the plurality of chemical samples corresponding to a different position in the plurality of positions, and each position in the plurality of positions representing a different period of growth of the corresponding biological sample associated with chemical dynamics, wherein the biological sample comprises a tooth sample, a nail sample, a hair sample, or any combination thereof, and wherein the plurality of positions comprises:
one or more positions representing a period of growth prior to the intervention,
one or more positions representing a period of growth during the intervention, and
one or more positions representing a period of growth after the intervention;
analyzing, for each respective subject in the plurality of subjects, the corresponding plurality of chemical samples for the respective subject thereby obtaining a corresponding first dataset that includes a plurality of traces, each trace in the plurality of traces being a concentration of a corresponding chemical, in a plurality of chemicals, over time collectively determined from the plurality of chemical samples,
applying, for each respective subject in the plurality of subjects and for each chemical in the plurality of chemicals, a distributed lag model as a function of time relative to the intervention to the concentration of the respective chemicals measured from the plurality of positions, to generate a corresponding contribution data set representing the contribution of the intervention to the concentration of the respective chemicals in the respective subject as a function of time; and
evaluating changes in chemical dynamics in response to the intervention using the corresponding contribution data set, for each of the one or more chemicals, from each respective subject in the plurality of subjects
wherein the intervention comprises an environmental input, a pharmaceutical compound, a diet, a supplement, a nutraceutical, or any combination thereof, for each respective subject of the plurality of subjects participating in a clinical trial, a community trial, or any combination thereof.

### Intervention Optimization

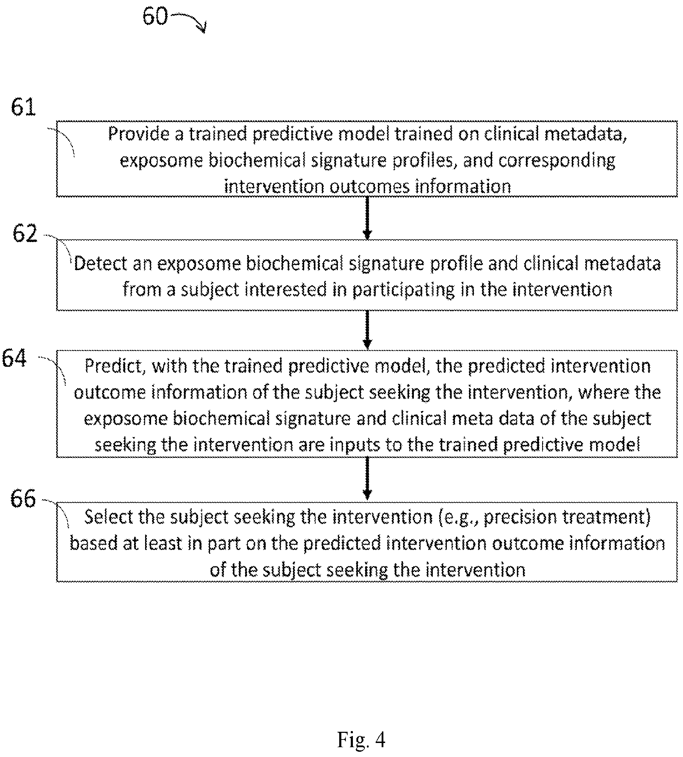

In some embodiments, the methods of the disclosure may comprise a method of optimizing the outcome of intervention for subjects **60,** as seen in **FIG. 4****.** In some embodiments, intervention may comprise clinical trial studies at phase I, phase II, phase III, or any combination thereof intervention. In some embodiments, the method comprises the steps of: (a) providing a trained predictive model, where the trained predictive model is trained on one or more subjects' clinical metadata, exposomic features, and corresponding intervention outcome information **61;** (b) detecting features derived from a biochemical signature obtained from a biological sample from a subject seeking the intervention, thereby producing a retrospective exposome biochemical signature **62;** (c) predicting, with the trained predictive model, the predicted intervention outcome information of the subject seeking the intervention, where the retrospective exposome biochemical signature profile and clinical meta of the subject seeking the intervention are inputs to the trained predictive model **64;** (d) selecting the subject for the intervention or excluding the subject from the intervention, based at least in part on the predicted intervention outcome information of the subject **66.** Alternatively, the intervention may comprise a community trial that may or may not be performed in a clinical setting.

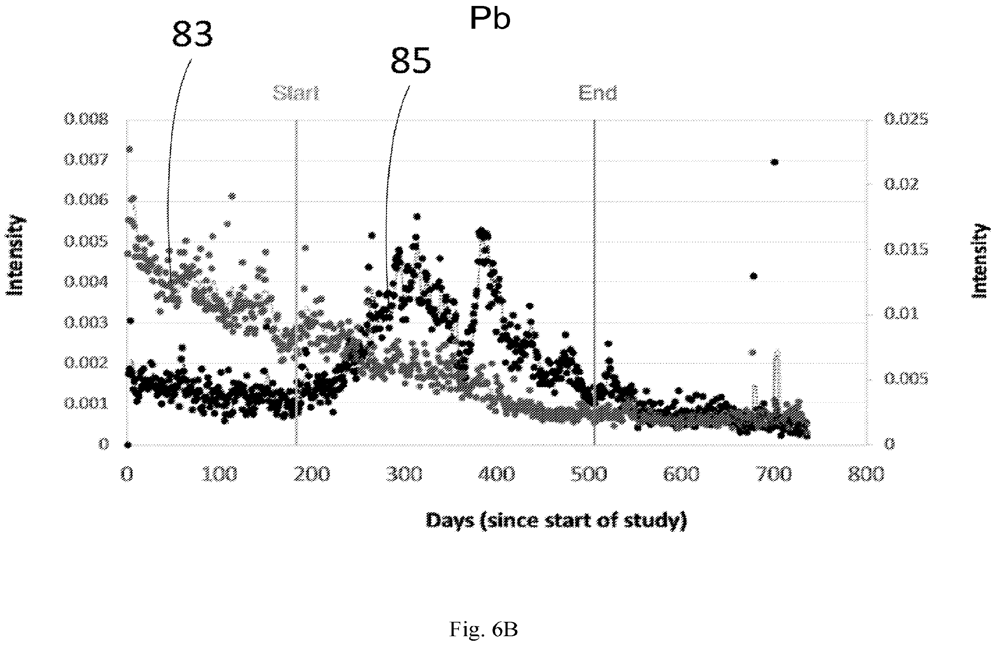

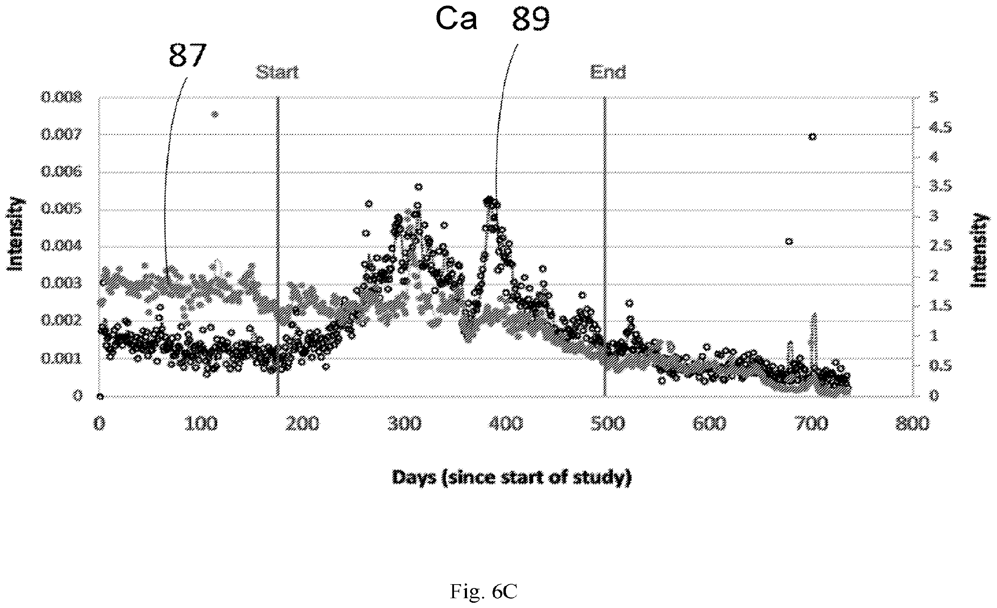

In some instances, subjects' one or more exposomic features may be used to determine the effectiveness or efficacy of a given intervention. For example, as seen in **FIGS. 6A-D,** exposomic analysis of subjects' biological samples to generate exposomic features may provide insight into the effectiveness of a lead-based poisoning intervention. **FIG.** 6A shows an exposome biochemical signature **81** with the x-axis of days and y-axis of exposome signature intensity. **FIG. 6A** represents the exposome signature of tin with start 77 and end 79 points of the intervention indicated. **FIG. 6B-D** show contrasting exposome biochemical signatures for exposome signatures of lead, calcium and magnesium, respectively, for one or more subjects that did not receive the intervention **85, 89,** and **93** compared to subjects that did receive the intervention **83, 87,** and **91.** For this particular example, the exposome biochemical signature for lead in **FIG. 6C** may be observed to decrease, indicating that the intervention may have proven to be effective. However, it may also be observed that other exposome signatures such as magnesium increase potentially leading to unwanted effects. In some embodiments, such exposomic features of the biochemical signature are utilized to observe the effectiveness of an intervention, or to recommend adjunctive intervention to supplement indications of unwanted increases or decreases in one or more exposomic features that are not the target of the intervention. In some embodiments, such an approach of intervention efficacy or effectiveness analysis is used to repurpose interventions for non-intended applications or to aid in symptoms from diseases or disorders that the intervention was not initially intended for.

In some embodiments, exposomic features acquired from subjects receiving interventions are further analyzed with a plurality of analytical modules. In some embodiments, the first analytical module (Module 1) focuses on the effect of clinical intervention on elemental signal intensities where the time-course of intervention is established relative to the timing of exposome biochemical signature signal intensities. Time-varying signal intensities for exposome biochemical signatures may be dated to the timing of clinical intervention, allowing exposome biochemical signature signal intensities to be delineated as occurring prior to intervention, concurrent to intervention, or following an intervention. Exposome biochemical signature signal intensities during these periods can be aggregated at the level of the subject via summary statistics such as the mean or median exposome biochemical signature signal intensity detected during that period. The effect of intervention can then be assessed across subjects participating in a study through the application of traditional general linear models, where exposome biochemical signature signal intensities prior to and following intervention are compared across all subjects in order to identify statistically significant differences in exposome biochemical signature signal intensity corresponding to the effects of intervention. In this context statistical significance is evaluated through standard probabilistic hypothesis testing.

In some embodiments, the second analytical module (Module 2) may comprise a focus on the simultaneous effects of an intervention on multiple exposomic features i.e., biochemical signature pathways. As in Module 1, this module may be applied when the time-course of intervention is established relative to the timing of exposome biochemical signature signal intensities, thereby allowing the aggregation of pre-intervention, intervention-concurrent, and post-intervention intensities with descriptive statistics. Aggregate measures derived at the level of the individual are then pooled across participants in the clinical trial and used in the construction of multivariate models. These may take the form of unsupervised analyses, such as principle component analysis, factor analysis, or related methods, whereby a dimensionality-reduction technique is applied to derive metrics (principle components; factor scores) which summarize exposome biochemical signature signal intensities for multiple exposome biochemical signature pathways, which can then be used in subsequent general linear models to test hypotheses relating to clinical intervention, as in Module 1. Alternatively, a supervised-dimensionality reduction technique, such as partial least squares, partial least squares discriminant analysis, linear discriminant analysis, weighted quantile sum regression, or Bayesian kernel machine regression may be used to directly link the effect of intervention to changes in exposome biochemical signature signal intensities across multiple exposome biochemical signatures.

In some embodiments, a third analytical module (Module 3) is used in circumstances when the exact timing of an intervention is unknown, or in embodiments where the effect of the intervention is expected to have a time-lagged effect; for example, if changes in exposome biochemical signature signal intensities do not manifest for some time after treatment, or if the timing of treatment-evoked change varies among individuals. In these cases, modeling strategies derived from econometrics may be used; in particular, the implementation of distributed lag models and related non-linear methods. These methods may be extended, as in Module 2, to include the simultaneous evaluation of intervention effects in multiple exposome biochemical signatures , for example through the implementation of lagged weighted quantile sum regression. Alternative analytical strategies may comprise the use of change-point detection methods via moving average methods, self-exciting threshold autoregressive (SETAR) models, autoregressive moving average models (ARMA), bayesian change-point detection, and related methodologies, particularly relating to longitudinal modeling and change-point detection.

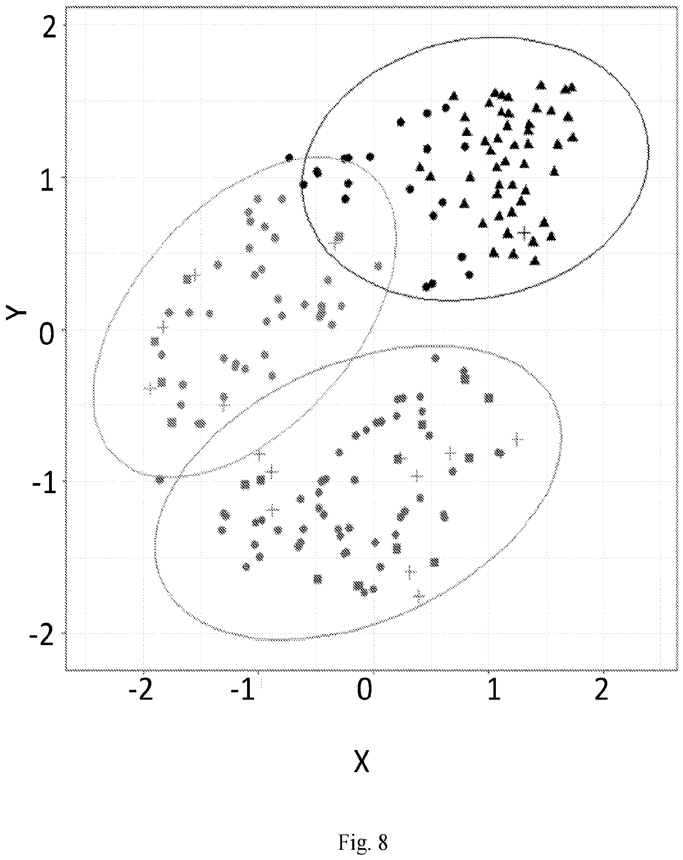

In some embodiments, a fourth analytical module (Module 4), unlike the prior models, may focus on the analysis of signal dynamics derived from analysis of longitudinal biochemical signature profile signals. Signal dynamics in this context refers to parameters derived from the analysis of biochemical signature profile signal intensities, which may include estimation of parameters descriptive of underlying processes such as periodicity, entropy, and stationarity. One approach to achieve this may comprise the application of recurrence quantification analysis (RQA) to individual biochemical signature profile signals measured in each subject trial participant. For each longitudinal measurement of a given biochemical signature profile pathway, the application of RQA may yield multiple quantitative measures or features, including any combination of recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, and number of the most probable recurrences. These features may be extracted from multiple biochemical signature pathways, and from the analysis of interactions among pathways via cross recurrence quantification analysis (CRQA), for use in subsequent analysis, where features derived from each subject are pooled and used to test for study-wide effects relating to the intervention. This approach may be particularly applicable to case-control study designs where some subjects received placebo treatment, and the interest is in distinguishing differences in elemental signal dynamics between intervention and placebo experimental conditions. In such contexts, the parameters derived from RQA/CRQA may be tested in a traditional analytical framework, for example via general linear models, in order to evaluate difference in signal parameters between treatment conditions. This approach may also be applicable to cases similar to those described in modules 1 and 2, where the time-course of a given treatment may is known and the interest is in distinguishing pre-treatment, treatment-concurrent, and post-treatment conditions. In this context RQA/CRQA may be applied either to subsets of the full elemental trace, corresponding to pre-treatment, treatment-concurrent, and post-treatment conditions; or, a variant of RQA/CRQA, utilizing a windowed binning technique, may be utilized to derive a longitudinal measure of RQA/CRQA parameters, which may subsequently be analyzed with methods described in Module 1,2, or 3. In any combination of these conditions, the features derived from dynamical signal analysis via RQA and related methods can also be used in supervised and unsupervised dimensionality reduction techniques, as described in Module 2, for the sub-typing of subjects on the basis of biochemical signature profiles. These approaches can be used prior to interventions, towards the goal of identifying patient/participant subtypes, or can be used subsequent to treatment, in order to link the effect of clinical intervention to associated metabolic pathways. As can be seen in **FIG. 8****,** hair samples provided by subjects' with autism spectrum disorder (ASD) were analyzed via this method. The resulting projection illustrates the derivation of three ASD sub-types from the analysis of biochemical signature profiles- in this case, by application of k-means clustering to RQA of elemental profiles. The specification of subject type can thereafter be used in subsequent clinical analyses and decision-making.

### Pharmaceutical and Nutraceutical Recommendation

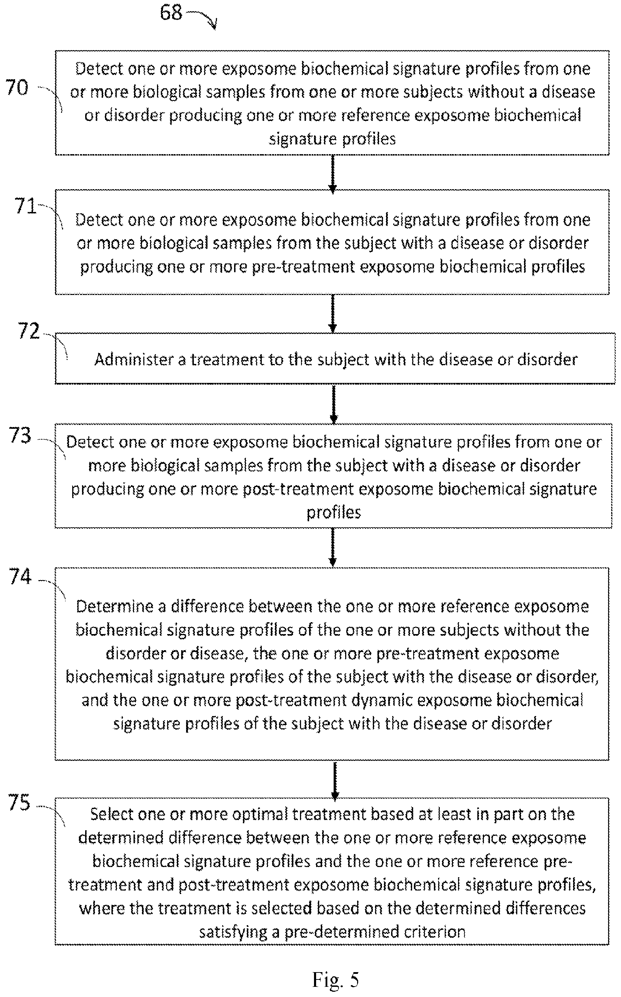

In some embodiments, the methods of the disclosure may comprise a method of optimal pharmaceutical and or nutraceutical recommendations for subjects suffering from a disease or disorder **68,** as seen in **FIG.** 5. In some embodiments, the method may comprise the steps of: (a) detecting features derived from one or more biochemical signatures obtained from one or more biological sample from one or more subjects without the disease or disorder, thereby producing a one or more reference exposomic features **70;** (b) detecting features derived from one or more biochemical signature obtained from one or more biological sample from the subject with the disease or disorder, thereby producing one or more features of pre-treatment exposomic features **71;** (c) administering a treatment to the subject with the disease or disorder **72;** (d) detecting one or more exposomic features obtained from one or more biological samples from one or more subjects with the disease or disorder after a period of time has elapsed after receiving the treatment, thereby producing one or more post-treatment exposomic features **73;** (e) determining a difference between the one or more features of the reference exposomic features of the one or more subjects without the disorder or disease, features of the one or more pre-treatment exposomic features of the one or more subjects with the disease or disorder, and features of the one or more post-treatment exposomic features of the one or more subjects with the disease or disorder **74;** and (f) selecting one or more optimal treatments based at least in part on the determined difference between the features of one or more reference exposomic features, one or more pre-treatment exposome exposomic features and one or more post-treatment exposomic features, where the one or more optimal treatments are selected based on the determined differences satisfying a pre-determined criterion 75.

In some embodiments, determining the difference between the one or more reference exposomic features of the one or more subjects without the disorder or disease, the one or more pre-treatment exposomic features of the one or more subjects with the disease or disorder, and the one or more post-treatment exposomic features of the one or more subjects with the disease or disorder may be implemented by means of predicative models described elsewhere herein.

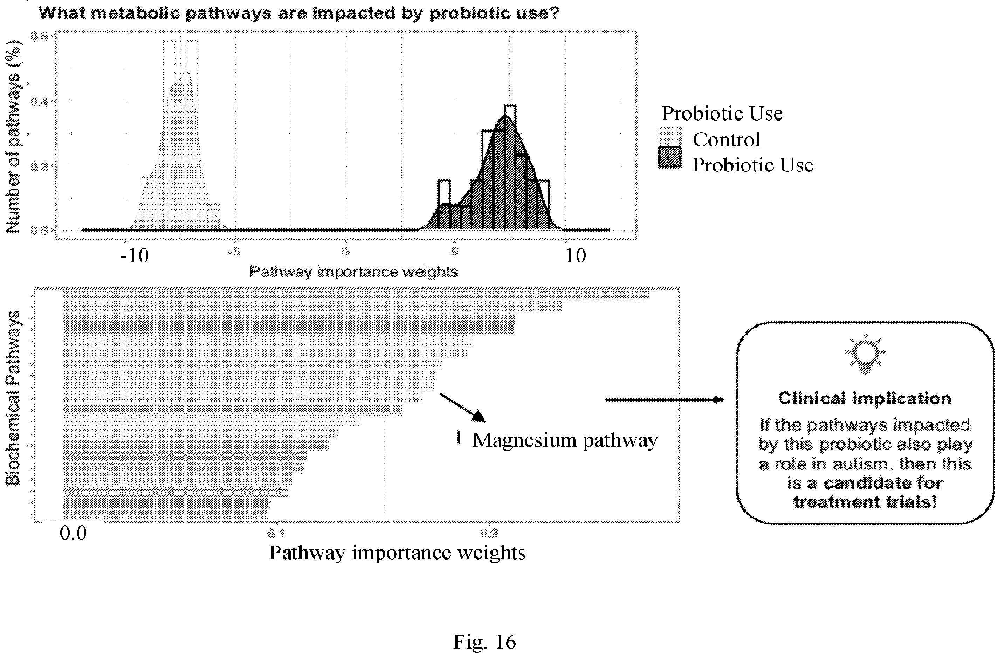

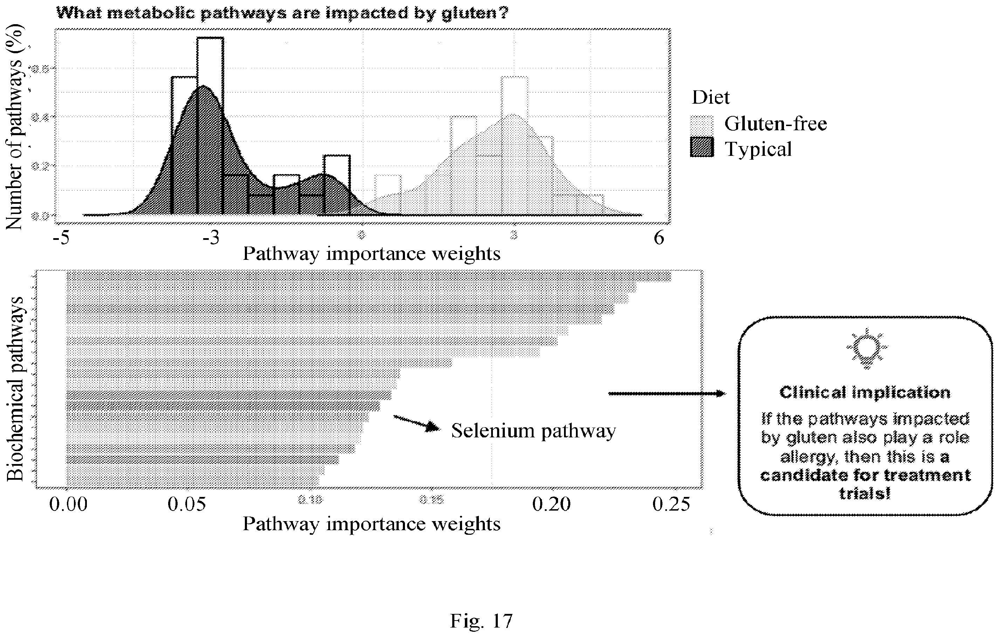

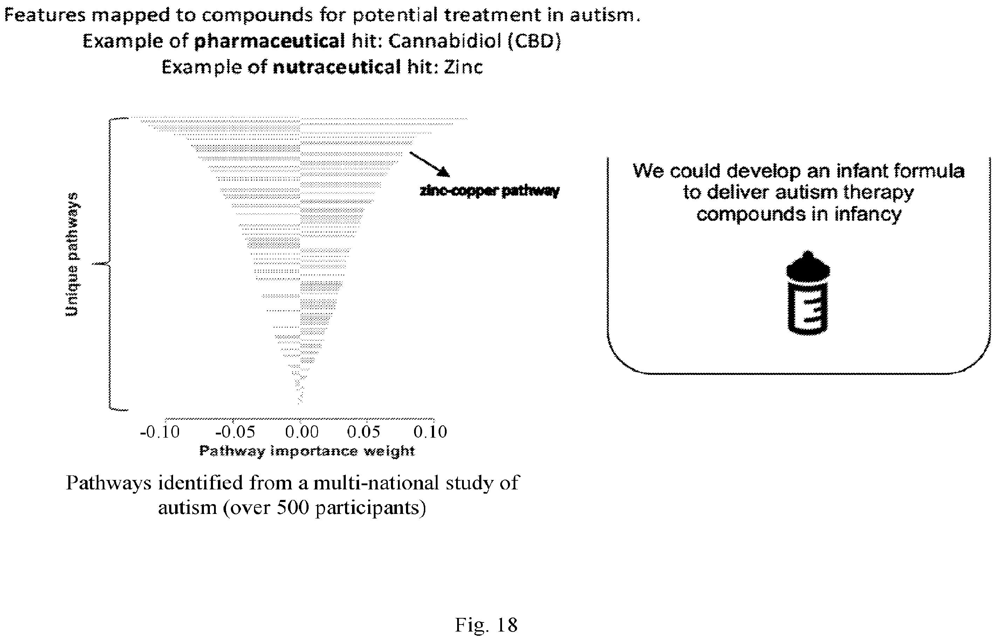

In some embodiments, the methods of optimal pharmaceutical and/or nutraceutical recommendation for subjects is used to analyze the influence and/or importance of exposome biochemical signature pathways impacted by intervention (e.g., pharmaceutical and/or nutraceuticals), as seen in **FIGS. 16-18****.** In some instances, the features of the exposome biochemical signature pathways of an intervention and a control group may be compared. In some embodiments the intervention comprises probiotic use **(****FIG. 16****),** a gluten free diet **(****FIG. 17****),** cannabidiol, zinc **(****FIG. 18****),** or any combination thereof. In some instances, the intervention may be delivered as infant formula **(****FIG. 18****).** The outcome of the features of the exposome biochemical signature pathway analysis may comprise a recommended intervention with exposome biochemical signature pathways complementary or otherwise impacted in a similar manner with a disease or disorder. In some embodiments, the disease or disorder comprises psychological, cardiac, gastroenterological, pulmonary, neurological, circulatory, nephrological, or any combination thereof disease or disorders. In some embodiments, the disease or disorder comprises cancer, e.g., pediatric cancer, lung cancer, etc. In some embodiments the disease or disorder comprises, for example, autism spectrum disorder (ASD), attention deficit hyperactivity disorder (ADHD), amyotrophic lateral sclerosis (ALS), schizophrenia, irritable bowel disease (IBD), pediatric kidney disease, kidney transplant rejection, pediatric cancer or any combination thereof.

### Determining Health Outcomes

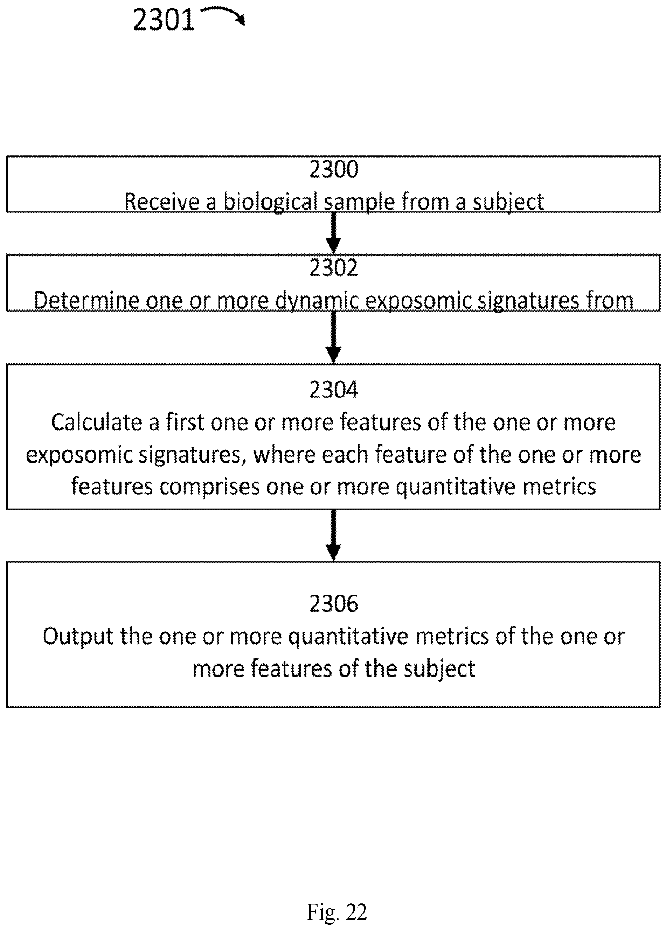

In some embodiments, the disclosure describes a method for outputting one or more quantitative metrics of a subject's one or more dynamic exposomic features **2301,** as seen in **FIG. 22****.** The method may comprise the following operations: (a) receiving a biological sample from a subject **2300;** (b) determining one or more dynamic exposomic signatures from the biological sample of the subject **2302;** (c) calculating a first one or more features of the one or more dynamic exposomic signatures, where each feature of the one or more features comprises one or more quantitative metrics **2304;** and (d) outputting the one or more quantitative metrics of the one or more features of the subject **2306.**

In some embodiments, the method further comprises outputting a health outcome of the subject based at least in part on an association of normalized scores of the subject's one or more features to normalized scores of a second set of subjects' one or more features' one or more quantitative metrics. In some instances, the second set of subjects' one or more features' one or more quantitative metrics may be stored in a database, where the database is a local server or a cloud-based server. In some instances, the health outcome may comprise a diagnosis of disease state, disease subtype, clinical subtype, non-clinical subgrouping related to physiology, anthropometric indicators, behavior indicators, socioeconomic indicators, body mass index, intelligence quotient, socio-economic status, or any combination thereof. In some embodiments, the one or more features comprise a measurement of temporal dynamics of the one or more dynamic exposomic signatures.

In some instances, the measurement of the temporal dynamics may comprise: linear slope, non-linear parameters describing curvature of the one or more dynamic exposomic signatures, abrupt changes in intensity of the one or more dynamic exposomic signatures, changes in baseline intensity of the one or more dynamic exposomic signatures, changes of the frequency-domain representation of the one or more dynamic exposomic signatures, changes of the power-spectral domain representation of the one or more dynamic exposomic signatures, recurrence quantification analysis parameters, cross-recurrence quantification analysis parameters, joint recurrence quantification analysis parameters, multidimensional recurrence quantification analysis parameters, estimation of the lypanuv spectra, maximum Lyapunov exponent, or any combination thereof. In some embodiments, the disease state comprises: autism spectrum disorder (ASD), attention deficit hyperactivity disorder (ADHD), amyotrophic lateral sclerosis (ALS), schizophrenia, irritable bowel disease (IBD), pediatric kidney disease, kidney transplant rejection, cancer, or any combination thereof.

In some instances, the one or more features of the one or more dynamic exposomic signatures may comprise phenotypic features, where the phenotypic features comprise: electrocardiogram (ECG), electroencephalography, magnetic resonance imaging (MRI), functional magnetic resonance imaging (fMRI), positron emission tomography (PET), genomic, epigenomic, transcriptomic, proteomic, metabolomic, or any combination thereof data. In some embodiments, the one or more features are derived from one or more attractors. In some instances, the one or more dynamic exposomic signatures may be measured by mass spectrometry, laser ablation-inductively coupled plasma mass spectrometry, laser induced breakdown spectroscopy, Raman spectroscopy, immunohistochemistry fluorescence, or any combination thereof. In some instances, the biological sample may comprise hair, teeth, toenails, finger nails, physiologic parameters, or any combination thereof. In some instances, the phenotypic features may comprise molecular phenotypes. In some instances, the molecular phenotypes may be determined by unsupervised analysis, where unsupervised analysis may comprise clustering, dimensionality-reduction, factor analysis, stacked autoencoding, or any combination thereof.

In some embodiments, the recurrence quantification analysis parameters comprise recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, and number of the most probable recurrences. In some embodiments, the method further comprises analyzing the one or more attractors by potential energy analysis thereby producing a potential energy data space. In some instances, the subject's one or more dynamic exposomic signatures may comprise retrospective, prospective, or any combination thereof dynamic exposomic data. In some instances, the method may further comprise analyzing a dynamic relationship between the one or more attractors' signal recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, or any combination thereof. In some embodiments, the dynamic relationship is determined by cross-convergent mapping (CCM). The method of claim 1, further comprising reducing the one or more dynamic exposomic signatures to a reduced one or more exposomic dynamic signatures. In some instances, the method may further comprise constructing a network of the one or more attractors based on similarity of the one or more attractors' temporal exposomic data signal recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, number of the most probable recurrences, or any combination thereof. In some instances, the method may further comprise analyzing one or more features of the network of the one or more attractors to determine network connectivity, efficiency, feature importance, pathway importance, related graph-theory based metrics, or any combination thereof.

### Predicting Phenotypic Data.

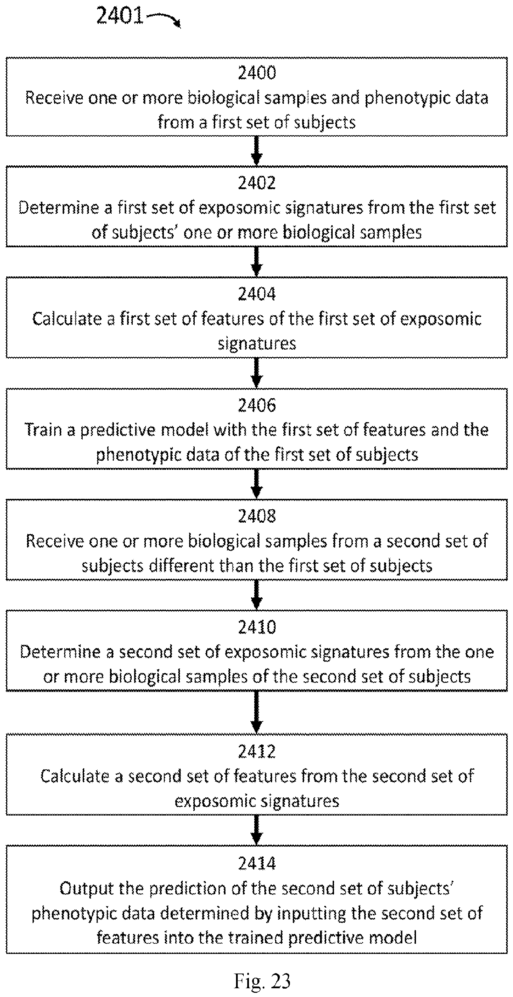

In some embodiments, the disclosure describes a method for outputting a prediction of one or more subjects' phenotypic data **2401,** as seen in **FIG. 23****.** The method may comprise the following operations: (a) receiving one or more biological samples and phenotypic data from a first set of subjects **2400;** (b) determining a first set of exposomic signatures from the first set of subjects' one or more biological samples **2402;** (c) calculating a first set of features of the first set of exposomic signatures **2404;** (d) training a predictive model with the first set of features and the phenotypic data of the first set of subjects **2406;** (e) receiving one or more biological samples from a second set of subjects different than the first set of subjects **2408;** (f) determining a second set of exposomic signatures from the one or more biological samples of the second set of subjects **2410;** (g) calculating a second set of features from the second set of exposomic signatures **2412;** and (h) outputting the prediction of the second set of subjects' phenotypic data determined by inputting the second set of features into the trained predictive model **2414.**

In some embodiments, the first and second set of features comprise one or more quantitative metrics. In some instances, the one or more quantitative metrics may comprise a measurement of temporal dynamics of the first and second set of exposomic signatures. In some instances, the measurement of the temporal dynamics may comprise: linear slope, non-linear parameters describing curvature of the first and second set of exposomic signatures, abrupt changes in intensity of the first and second set of exposomic signatures, changes in baseline intensity of the first and second set of exposomic signatures, changes of the frequency-domain representation of the first and second set of exposomic signatures, changes of the power-spectral domain representation of the first and second set of exposomic signatures, recurrence quantification analysis parameters, cross-recurrence quantification analysis parameters, joint recurrence quantification analysis parameters, multidimensional recurrence quantification analysis parameters, estimation of the lypanuv spectra or maximum Lyapunov exponent, or any combination thereof.

In some embodiments, the first and second set of features comprise phenotypic features, where the phenotypic features may comprise a disease state or a healthy state, where the disease state comprises: autism spectrum disorder (ASD), attention deficit hyperactivity disorder (ADHD), amyotrophic lateral sclerosis (ALS), schizophrenia, irritable bowel disease (IBD), pediatric kidney disease, kidney transplant rejection, cancer, or any combination thereof. In some instances, the phenotypic features may further comprise: electrocardiogram (ECG), electroencephalography, magnetic resonance imaging (MRI), functional magnetic resonance imaging (fMRI), positron emission tomography (PET), genomic, epigenomic, transcriptomic, proteomic, metabolomic, or any combination thereof data.

In some instances, the first and second set of features may be represented as or derived from one or more attractors. In some embodiments, the one or more attractors is a limit cycle attractor, bistable attractor, or any combination thereof. In some embodiments, the first and second set of exposomic signatures are measured by mass spectrometry, laser ablation-inductively coupled plasma mass spectrometry, Raman spectroscopy, immunohistochemistry fluorescence, or any combination thereof. In some instances, the one or more biological sample of the first and second subjects may comprise hair, teeth, toenails, finger nails, physiologic parameters, or any combination thereof.

In some embodiments, the phenotypic features comprise molecular phenotypes. In some instances, the molecular phenotypes may be determined by unsupervised analysis, where unsupervised analysis comprises clustering, dimensionality-reduction, factor analysis, stacked autoencoding, or any combination thereof. In some instances, the recurrence quantification analysis parameters may comprise recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, and number of the most probable recurrences, or any combination thereof.

In some instances, the method may further comprise analyzing the one or more attractor by potential energy analysis thereby producing a potential energy data space. In some embodiments, the first and second set of exposomic signatures comprises retrospective, prospective, or any combination thereof exposomic data.

In some embodiments, the method further analyzes a dynamic relationship between the one or more attractors' recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, a number of the most probable recurrences, or any combination thereof. In some embodiments, the dynamic relationship is determined by cross-convergent mapping (CCM).

In some instances, the method may further comprise constructing a network of the one or more attractors based on similarity of the one or more attractors' temporal exposomic data signal recurrence rates, determinism, mean diagonal length, maximum diagonal length, divergence, Shannon entropy in diagonal length, trend in recurrences, laminarity, trapping time, maximum vertical line length, Shannon entropy in vertical line lengths, mean recurrence time, Shannon entropy in recurrence times, and number of the most probable recurrences, or any combination thereof. In some embodiments, the method further comprises analyzing one or more features of the network of the one or more attractors to determine network connectivity, efficiency, feature importance, pathway importance, related graph-theory based metrics feature importance, pathway importance, or any combination thereof.

Although the above steps show each of the methods or sets of operations, a person of ordinary skill in the art will recognize many variations based on the teaching described herein. The steps may be completed in a different order. Steps may be added or omitted. Some of the steps may comprise sub-steps. Many of the steps may be repeated as often as beneficial.

One or more of the steps of each of the methods or sets of operations may be performed with circuitry as described herein, for example, one or more of the processor or logic circuitry such as programmable array logic for a field programmable gate array. The circuitry may be programmed to provide one or more of the steps of each of the methods or sets of operations described elsewhere herein and the program may comprise program instructions stored on a computer readable memory or programmed steps of the logic circuitry such as the programmable array logic or the field programmable gate array, for example.

### EXAMPLES

### Example 1: Acquiring Exposomic Biochemical Signatures from A Biological Sample.

Temporal exposome biochemical signatures were acquired from a biological sample using laser ablation-inductively coupled-plasma mass spectrometry (LA-ICP-MS). For this particular example, hair shaft samples were used and chemicals exposome data was acquired. The hair shaft was harvested from subjects and pretreated by washing the sample with one or more solvents and/or surfactants followed by drying. Particularly, the hair shaft sample was washed in TRITON X-100 and ultrapure metal free water (*e.g*., MILLI-Q water) and dried overnight in an oven *(e.g.,* at 60 °C). The pretreatment further includes preparing the hair shaft for measurement by placing the hair shaft on a glass slide (*e.g*., a microscopic glass slide) with an adhesive film (*e.g.*, a double-sided tape). The hair shaft is positioned such that the hair shaft is substantially straight. The glass slide with the hair shaft is then placed into a LA-ICP-MS system for analysis.

The analysis commences by the LA-ICP-MS system completing a pre-ablating step where the hair shaft sample is ablated to remove surface debris and/or impurities from the hair shaft. The pre-ablation is performed with a low laser energy, approximately 10% laser energy that only releases particles on the surface of the hair shaft sample but does not release particles from below the surface. The pre-ablation is performed using a laser wavelength of 193 nm and a laser energy below 0.6 J/cm² (e.g., the laser energy is 0.6 J/cm2, 0.5 J/cm2, 0.4 J/cm², 0.3 J/cm², 0.2 J/cm², or 0.1 J/cm²).

After pre-ablation, the LA-ICP-MS system irradiates the hair shaft sample with a laser energy below 1.8 J/cm2 with laser spot size of 10 µm to 30µm to obtain chemical samples from respective positions along a reference line of the hair shaft sample. Each position along the hair shaft approximates to 2.2 hours or 130 minutes of time of the subject's life. The laser irradiating spatially irradiates the entirety of length of the hair shaft producing particles at a discretion location of the hair shaft that are then ionized with an inductively coupled plasma. The mass spectrometer then analyzes the obtained chemical samples providing the respective chemicals data read-out of what chemicals are present in what quantities at a given spatial location. This process is repeated, and chemicals data is collected sequentially at a plurality of positions along the hair shaft from the hair shaft root to the tip of the shaft furthest away from the root. Positional data, indicating time, and the respective isotopes present at each location of the hair shaft are correlated for further analysis.

The output data is further analyzed and processed by spike removal that removes extreme peaks in the exposome biochemical signature and smoothens the data. Outliers are identified by calculating the mean absolute difference between each adjacent measurement along the hair shaft. Values indicating a mean absolute difference from the preceding point exceeds three standard deviations of the mean are flagged as outliers. These outlier values are then replaced with a moving median filter, which calculates a running median value of the original exposome biochemical signature in bins of 10 adjacent data points.

Processed data can then be used in various bioinformation analysis methods that identify the effects of clinical intervention on elemental signal intensities and signal dynamics.

### Example 2: Dynamic molecular profiles in tooth samples for determining disease risk.

Using methods and systems of the present disclosure, molecular profiles in tooth samples were generated and subsequently analyzed to determine a disease risk in a subject. Generally, the temporal dynamics of biological response (e.g., inflammation) were found to be imprinted in samples (e.g., tooth samples), and can be analyzed to determine disease risk in a subject. Dynamic molecular profiles were generated for C-reactive protein (CRP), which is a marker of inflammation. Using the tooth biomarkers, dynamic time-series profiles of CRP and inflammation were generated during a time period that comprised fetal (prenatal) development and early childhood in two sets of children-a first set with autism spectrum disorder (37 cases) and a second set without autism spectrum disorder (77 controls). The time-series CRP profiles were analyzed to reveal novel features of the dynamics of the CRP signal, which accurately distinguished the autism cases from controls. For example, the inflammation profiles that were present before age of 1 year were highly differential between cases and controls. In comparison, a clinical diagnosis of autism is usually determined around the age of 3 to 4 years.

A primary tooth sample was obtained from each child subject. The teeth samples were sectioned open, decalcified and an immunohistochemistry stain (e.g., dentine) was applied to the teeth samples. The immunohistochemistry stain effectively mapped C-reactive protein (a molecular marker of inflammation) along the growth rings of the teeth samples in order to develop temporal profiles of inflammation over the prenatal and postnatal period. The temporal profiles were analyzed using machine learning algorithms of the present disclosure to train highly accurate classifiers to determine disease risk (e.g., autism).

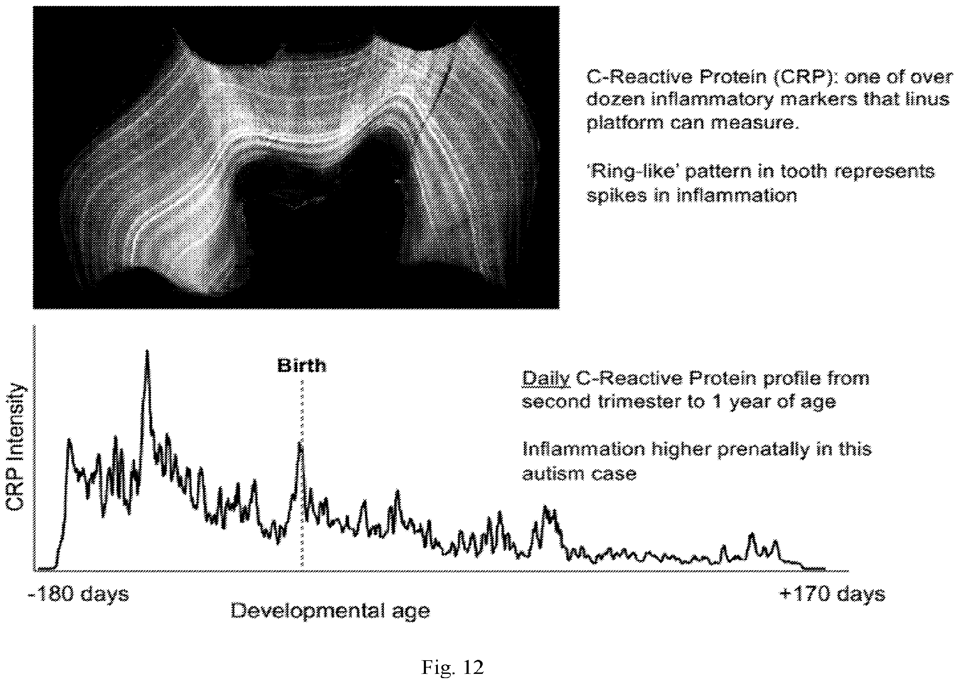

**FIG. 12** shows an example of a daily C-reactive protein profile of a subject over time, where the y-axis is indicative of CRP intensity and the x-axis is indicative of developmental age. The developmental age of the child subject included a time period ranging from the second trimester of gestation (e.g., starting at 140 days before birth, when the subject was in the prenatal stage) to about 6 months of age. As shown in **FIG. 12****,** inflammation (as indicated by CRP intensity) profile of a child with autism with high CRP intensity prenatally.

## Claims

1. A method for evaluating the effects of an intervention in a plurality of subjects, the method comprising:
sampling, for each respective subject in the plurality of subjects, each respective position in a plurality of positions along a reference line on a corresponding biological sample associated with chemical dynamics of the respective subject, thereby obtaining a corresponding plurality of chemical samples for the respective subject, each chemical sample in the plurality of chemical samples corresponding to a different position in the plurality of positions, and each position in the plurality of positions representing a different period of growth of the corresponding biological sample associated with chemical dynamics, wherein the biological sample comprises a tooth sample, a nail sample, a hair sample, or any combination thereof, and wherein the plurality of positions comprises:
one or more positions representing a period of growth prior to the intervention,
one or more positions representing a period of growth during the intervention, and
one or more positions representing a period of growth after the intervention;
analyzing, for each respective subject in the plurality of subjects, the corresponding plurality of chemical samples for the respective subject thereby obtaining a corresponding first dataset that includes a plurality of traces, each trace in the plurality of traces being a concentration of a corresponding chemical, in a plurality of chemicals, over time collectively determined from the plurality of chemical samples,
applying, for each respective subject in the plurality of subjects and for each chemical in the plurality of chemicals, a distributed lag model as a function of time relative to the intervention to the concentration of the respective chemicals measured from the plurality of positions, to generate a corresponding contribution data set representing the contribution of the intervention to the concentration of the respective chemicals in the respective subject as a function of time; and
evaluating changes in chemical dynamics in response to the intervention using the corresponding contribution data set, for each of the one or more chemicals, from each respective subject in the plurality of subjects
wherein the intervention comprises an environmental input, a pharmaceutical compound, a diet, a supplement, a nutraceutical, or any combination thereof, for each respective subject of the plurality of subjects participating in a clinical trial, a community trial, or any combination thereof.

2. The method of claim 1, wherein the intervention is ingestion of a nutraceutical composition.

3. The method of claim 2, further comprising, in response to the evaluating changes in chemical dynamics, altering the composition of the nutraceutical composition to adjust the effects of the ingestion of the nutraceutical composition.

4. The method of claim 2, further comprising evaluating changes in a metabolism of one or more metabolites in response to the intervention.

5. The method of claim 4, wherein the one or more metabolites comprise a perfluoro compound, a paraben, a phthalate, a lipid, an amino acid, an amino acid derivative, a peptide, or any combination thereof.

6. The method of claim 1, wherein sampling comprises performing laser ablation-inductively coupled plasma mass spectrometry, laser induced breakdown spectroscopy, Raman spectroscopy, immunohistochemistry profiling, or any combination thereof.

7. The method of claim 1, wherein the period of growth after the intervention comprises at least 1 hour, at least 1 day, at least 1 week, at least 1 month, at least 1 year, or any combination thereof.

8. The method of claim 1, wherein the plurality of chemical comprises aluminum, arsenic, barium, bismuth, calcium, copper, iodide, lead, lithium, magnesium, manganese, phosphorus, sulfur, tin, strontium, zinc, or any combination thereof.

9. The method of claim 1, wherein the clinical trial comprises a clinical trial study at phase I, phase II, or phase III.

10. The method of claim 1, wherein the changes in chemical dynamics in response to the intervention are used at least in part to optimize selection criterion for one or more subjects different from the plurality of subjects to receive the intervention.

## Patentansprüche

1. Verfahren zur Bewertung der Auswirkungen einer Behandlung bei einer Vielzahl von Subjekten, das Verfahren umfassend:
Entnahme von Proben für jedes einzelne Subjekt aus der Vielzahl von Subjekten an jeder einzelnen Position aus einer Vielzahl von Positionen entlang einer Referenzlinie an einer entsprechenden biologischen Probe, die mit der chemischen Dynamik des jeweiligen Subjekts in Zusammenhang steht, wodurch eine entsprechende Vielzahl chemischer Proben für das jeweilige Subjekt gewonnen wird, wobei jede chemische Probe aus der Vielzahl chemischer Proben einer anderen Position aus der Vielzahl von Positionen entspricht und jede Position aus der Vielzahl von Positionen einen anderen Wachstumszeitraum der entsprechenden biologischen Probe darstellt, die mit der chemischen Dynamik in Zusammenhang steht, wobei die biologische Probe eine Zahnprobe, eine Nagelprobe, eine Haarprobe oder eine beliebige Kombination davon umfasst und wobei die Vielzahl von Positionen Folgendes umfasst:
eine oder mehrere Positionen, die eine Wachstumsphase vor der Behandlung darstellen,
eine oder mehrere Positionen, die eine Wachstumsphase während der Behandlung darstellen, und
eine oder mehrere Positionen, die eine Wachstumsphase nach der Behandlung darstellen;
Analysieren der entsprechenden Vielzahl chemischer Proben des jeweiligen Subjekts für jedes jeweilige Subjekt aus der Vielzahl von Subjekten, wodurch ein entsprechender erster Datensatz erhalten wird, der eine Vielzahl von Kurven enthält, wobei jede Kurve aus der Vielzahl von Kurven eine Konzentration einer entsprechenden Chemikalie aus einer Vielzahl von Chemikalien darstellt, die über die Zeit gemeinsam aus der Vielzahl chemischer Proben bestimmt wird,
Anwenden eines verteilten Verzögerungsmodells als Funktion der Zeit relativ zur Intervention auf die Konzentration der jeweiligen Chemikalien, die an den mehreren Zeitpunkten gemessen wurden, für jedes einzelne Subjekt aus der Vielzahl von Subjekten und für jede Chemikalie aus der Vielzahl von Chemikalien, um einen entsprechenden Beitragsdatensatz zu erzeugen, der den Beitrag der Behandlung zur Konzentration der jeweiligen Chemikalien bei den jeweiligen Subjekten als Funktion der Zeit darstellt; und
Auswerten von Veränderungen der chemischen Dynamik als Reaktion auf die Behandlung unter Verwendung des entsprechenden Beitragsdatensatzes für jede der einen oder mehreren Chemikalien bei jedem jeweiligen Subjekt aus der Vielzahl von Subjekten
wobei die Behandlung einen Umwelteinfluss, eine pharmazeutische Verbindung, eine Diät, ein Nahrungsergänzungsmittel, ein Nutrazeutikum oder eine beliebige Kombination davon für jedes jeweilige Subjekt aus der Vielzahl von Subjekten umfasst, die an einer klinischen Studie, einer Bevölkerungsstudie oder einer beliebigen Kombination davon teilnehmen.

2. Verfahren nach Anspruch 1, wobei die Behandlung die Einnahme einer nutrazeutischen Zusammensetzung ist.

3. Verfahren nach Anspruch 2, ferner umfassend, als Reaktion auf die Auswertung von Veränderungen der chemischen Dynamik, die Zusammensetzung der nutrazeutischen Zusammensetzung zu verändern, um die Wirkungen der Einnahme der nutrazeutischen Zusammensetzung anzupassen.

4. Verfahren nach Anspruch 2, ferner umfassend die Auswertung von Veränderungen im Stoffwechsel eines oder mehrerer Metaboliten als Reaktion auf die Behandlung.

5. Verfahren nach Anspruch 4, wobei der eine oder die mehreren Metaboliten eine Perfluorverbindung, ein Paraben, ein Phthalat, ein Lipid, eine Aminosäure, ein Aminosäurederivat, ein Peptid oder eine beliebige Kombination davon umfassen.

6. Verfahren nach Anspruch 1, wobei die Probenahme die Durchführung von Laserablations-induktiv gekoppelter Plasma-Massenspektrometrie, laserinduzierter Breakdown-Spektroskopie, Raman-Spektroskopie, immunhistochemischer Profilierung oder einer beliebigen Kombination davon umfasst.

7. Verfahren nach Anspruch 1, wobei die Wachstumsdauer nach der Behandlung mindestens 1 Stunde, mindestens 1 Tag, mindestens 1 Woche, mindestens 1 Monat, mindestens 1 Jahr oder eine beliebige Kombination davon umfasst.

8. Verfahren nach Anspruch 1, wobei die Vielzahl an chemischen Stoffen Aluminium, Arsen, Barium, Wismut, Kalzium, Kupfer, Iodid, Blei, Lithium, Magnesium, Mangan, Phosphor, Schwefel, Zinn, Strontium, Zink oder eine beliebige Kombination davon umfasst.

9. Verfahren nach Anspruch 1, wobei die klinische Studie eine klinische Studie der Phase I, Phase II oder Phase III umfasst.

10. Verfahren nach Anspruch 1, wobei die Veränderungen der chemischen Dynamik als Reaktion auf die Behandlung zumindest teilweise dazu verwendet werden, Auswahlkriterien für ein oder mehrere Subjekte zu optimieren, die sich von der Vielzahl von Subjekten unterscheiden, die die Behandlung erhalten sollen.

## Revendications

1. Procédé d'évaluation des effets d'une intervention sur une pluralité de sujets, le procédé comprenant :
l'échantillonnage, pour chaque sujet respectif parmi la pluralité de sujets, de chaque position respective parmi une pluralité de positions le long d'une ligne de référence sur un échantillon biologique correspondant associé à une dynamique chimique du sujet respectif, obtenant ainsi une pluralité correspondante d'échantillons chimiques pour le sujet respectif, chaque échantillon chimique parmi la pluralité d'échantillons chimiques correspondant à une position différente parmi la pluralité de positions, et chaque position parmi la pluralité de positions représentant une période de croissance différente de l'échantillon biologique correspondant associé à une dynamique chimique, dans lequel l'échantillon biologique comprend un échantillon de dent, un échantillon d'ongle, un échantillon de cheveux, ou toute combinaison de ceux-ci, et dans lequel la pluralité de positions comprend :
une ou plusieurs positions représentant une période de croissance antérieure à l'intervention,
une ou plusieurs positions représentant une période de croissance pendant l'intervention, et
une ou plusieurs positions représentant une période de croissance après l'intervention ;
l'analyse, pour chaque sujet respectif parmi la pluralité de sujets, de la pluralité correspondante d'échantillons chimiques pour le sujet respectif, obtenant ainsi un premier ensemble de données correspondant qui inclut une pluralité de traces, chaque trace parmi la pluralité de traces étant une concentration d'un produit chimique correspondant, parmi une pluralité de produits chimiques, déterminée collectivement au fil du temps à partir de la pluralité d'échantillons chimiques,
l'application, pour chaque sujet respectif parmi la pluralité de sujets et pour chaque produit chimique parmi la pluralité de produits chimiques, d'un modèle de décalage distribué en fonction du temps concernant l'intervention par rapport à la concentration des produits chimiques respectifs mesurée à partir de la pluralité de positions, pour générer un ensemble de données de contribution correspondant représentant la contribution de l'intervention par rapport à la concentration des produits chimiques respectifs dans le sujet respectif en fonction du temps ; et
l'évaluation de changements de dynamique chimique en réponse à l'intervention à l'aide de l'ensemble de données de contribution correspondant, pour chacun des un ou plusieurs produits chimiques, de chaque sujet respectif parmi la pluralité de sujets
dans lequel l'intervention comprend un apport environnemental, un composé pharmaceutique, un régime alimentaire, un complément, un nutraceutique, ou toute combinaison de ceux-ci, pour chaque sujet respectif de la pluralité de sujets participant à un essai clinique, un essai communautaire, ou toute combinaison de ceux-ci.

2. Procédé selon la revendication 1, dans lequel l'intervention est l'ingestion d'une composition nutraceutique.

3. Procédé selon la revendication 2, comprenant en outre, en réponse à l'évaluation des changements de dynamique chimique, l'altération de la composition nutraceutique pour ajuster les effets de l'ingestion de la composition nutraceutique.

4. Procédé selon la revendication 2, comprenant en outre l'évaluation de modifications chez un métabolisme d'un ou plusieurs métabolites en réponse à l'intervention.

5. Procédé selon la revendication 4, dans lequel les un ou plusieurs métabolites comprennent un composé perfluoro, un parabène, un phtalate, un lipide, un acide aminé, un dérivé d'acide aminé, un peptide, ou toute combinaison de ceux-ci.

6. Procédé selon la revendication 1, dans lequel l'échantillonnage comprend la réalisation d'une spectrométrie de masse à plasma à couplage inductif et ablation laser, d'une spectroscopie d'émission de plasma induit par laser, d'une spectroscopie Raman, d'un profilage immunohistochimique, ou toute combinaison de ceux-ci.

7. Procédé selon la revendication 1, dans lequel la période de croissance après l'intervention comprend au moins 1 heure, au moins 1 jour, au moins 1 semaine, au moins 1 mois, au moins 1 an, ou toute combinaison de ceux-ci.

8. Procédé selon la revendication 1, dans lequel la pluralité de produits chimiques comprend de l'aluminium, arsenic, baryum, bismuth, calcium, cuivre, iodure, plomb, lithium, magnésium, manganèse, phosphore, soufre, étain, strontium, zinc, ou toute combinaison de ceux-ci.

9. Procédé selon la revendication 1, dans lequel l'essai clinique comprend une étude d'essai clinique en phase I, phase II ou phase III.

10. Procédé selon la revendication 1, dans lequel les changements de dynamique chimique en réponse à l'intervention sont utilisés au moins en partie pour optimiser le critère de sélection pour un ou plusieurs sujets différents de la pluralité de sujets recevant l'intervention.
